# EUROPEAN PATENT SPECIFICATION

(11) **EP 1 505 109 B1**
(45) Date of publication and mention of the grant of the patent: **15.09.2010**
(21) Application number: 03725687.2
(22) Date of filing: 28.04.2003
(51) Int. Cl.: C08K 5/1575, C08L 23/00

(54) **AGENT FOR INHIBITING MIGRATION OF ODOR AND TASTE GENERATED FROM DIACETAL, DIACETAL COMPOSITION CONTAINING THE AGENT FOR INHIBITING ODOR AND TASTE MIGRATION, NUCLEATING AGENT FOR POLYOLEFIN COMPRISING THE COMPOSITION, AND POLYOLEFIN RESIN COMPOSITION AND MOLDED OBJECT EACH CONTAINING THE NUCLEATING AGENT**
MITTEL ZUM INHIBIEREN VON AUS EINEM DIACETAL ERZEUGTEM GERUCH ODER GESCHMACK, DIACETALZUSAMMENSETZUNG, DIE DAS MITTEL ZUM INHIBIEREN VON AUS EINEM DIACETAL ERZEUGTEM GERUCH ODER GESCHMACK ENTHÄLT, DIE ZUSAMMENSETZUNG ENTHALTENDES NUKLEIERUNGSMITTEL FÜR POLYOLEFIN; DAS NUKLEIERUNGSMITTEL ENTHALTENDE POLYOLEFINHARZZUSAMMENSETZUNG UND NUKLEIERUNGSMITTEL ENTHALTENDER FORMKÖRPER
AGENT PERMETTANT D'INHIBER LA MIGRATION D'ODEUR ET DE GOUT PROVOQUEE PAR LE DIACETAL, COMPOSITION DE DIACETAL CONTENANT CET AGENT PERMETTANT D'INHIBER LA MIGRATION D'ODEUR ET DE GOUT, AGENT DE NUCLEATION POUR POLYOLEFINE COMPRENANT CETTE COMPOSITION, ET COMPOSITION DE RESINE POLYOLEFINIQUE ET OBJET

(30) Priority: 02.05.2002 JP 2002130873
(43) Date of publication of application: 09.02.2005
(73) Proprietor: NEW JAPAN CHEMICAL CO., LTD., Kyoto-shi, Kyoto 612-8224 (JP)
(72) Inventor: ISHIKAWA, Masahide, Uji-shi, Kyoto 611-0002 (JP); UEOKA, Chiaki, Nara-shi, Nara 630-8141 (JP)
(74) Representative: Barz, Peter
(86) International application number: PCT/JP2003/005424
(87) International publication number: WO 2003/093360

(56) References cited:
- EP-A- 0 421 634
- EP-A- 0 522 558
- EP-A- 0 801 102
- EP-A2- 0 801 102
- JP-A- 8 217 924
- JP-A- 9 059 444
- JP-A- 9 157 452
- JP-A- 9 286 788
- DATABASE WPI Section Ch, Week 199803 Derwent Publications Ltd., London, GB; Class A17, AN 1998-029041 XP002450648 & JP 09 286788 A (NEW JAPAN CHEM. CO., LTD.) 4 November 1997 (1997-11-04)
- DATABASE WPI Section Ch, Week 198715 Derwent Publications Ltd., London, GB; Class A17, AN 1987-104180 XP002450649 & JP 62 050355 A (TOKUYAMA SODA K.K.) 5 March 1987 (1987-03-05)
- DATABASE WPI Section Ch, Week 198926 Derwent Publications Ltd., London, GB; Class A17, AN 1989-188417 XP002450650 & JP 01 126352 A (NEW JAPAN CHEM. CO., LTD.) 18 May 1989 (1989-05-18)

## Description

### TECHNICAL FIELD

The present invention relates to an agent for suppressing transfer of odor and taste originating from a diacetal, a diacetal composition comprising the suppressing agent, a polyolefin nucleating agent comprising the diacetal composition, a polyolefin resin composition comprising the nucleating agent, a polyolefin resin molded product prepared by molding the resin composition, and a method for suppressing transfer of odor and taste originating from a diacetal using the suppressing agent.

### BACKGROUND ART

Diacetals such as dibenzylidene sorbitol and nuclear-substituted derivatives thereof are useful compounds used as nucleating agents for polyolefin resins, specifically for homopolymers of ethylene or propylene, and copolymers predominantly comprising ethylene and propylene. Diacetals are especially effective in improving clarity, and widely used as resin additives in the field of molded products such as various containers requiring clarity.

However, the diacetals partly undergo thermal decomposition during processing, and release benzaldehydes constituting the diacetals to thereby emit an odor. For this reason, the aldehyde odor sometimes remains also in the final molded product. Moreover, when a molded product is used as a packaging material or a container for food a taste of aldehyde may transfer to the food in contact with this molded product. Therefore, the diacetals may not be preferred in the field of containers and packaging materials for foods and cosmetics.

There have been various proposals up to now aimed at ameliorating the above problems. Examples are treatment by a hydroxyamine or a phenylhydrazine (Japanese Unexamined Patent Publication No.1985-32791 and Japanese Unexamined Patent Publication No.1985-42385), addition of a non-aromatic organic amine (Japanese Unexamined Patent Publication No.1987-4289), surface treatment by an aliphatic metal salt or a lactic acid metal salt (Japanese Unexamined Patent Publication No.1987-50355), addition of an aliphatic amine (Japanese Unexamined Patent Publication No.1990-196841), addition of sorbic acid and/or potassium sorbate (Japanese Unexamined Patent Publication No.1993-202055), addition of an alkali metal salt of an amino acid (Japanese Unexamined Patent Publication No.1997-286787).

Nevertheless, the above prior art methods still have some drawbacks: the addition of said additives might cause yellowing of the polyolefin resin molded product; odor-improving effect is insufficient and limits application in the field of, e.g., food containers; and so on, and these and other problems have yet to be satisfactorily solved.

An object of the present invention is to provide a novel and useful polyolefin resin composition, in which aldehyde generation is significantly suppressed during processing and in the final molded product, thereby suppressing transfer of odor and taste.

JP-A-09286788 relates to a stabilized dibenzylidenesorbitol composition obtained by adding a polyol having at least two alcoholic groups at the 1- and 2-positions or at the 1- and 3-positions to the dibenzylidenesorbitol composition. The stabilized dibenzylidenesorbitol composition is incorporated into a polyolefin resin.

EP-A-0522558 describes a process for stabilizing dibenzylidenesorbitols comprising incorporating sorbic acid and/or potassium sorbate into the dibenzylidenesorbitol compound.

### DISCLOSURE OF THE INVENTION

Under the above circumstances, the inventors carried out intensive research to achieve the above object and found that when a diacetal composition comprising a diacetal and a specific long chain fatty alcohol or a specific hydroxycarboxylic acid (component (B)) is used as a nucleating agent for polyolefin, aldehyde generation is significantly suppressed in the resultant polyolefin resin pellets and the molded products thereof, thereby suppressing transfer of odor and taste.

In addition, it was found that a diacetal composition which further contains a specific anionic surfactant, a long chain fatty acid alkali metal salt or an amine (component (C)) in addition to the diacetal composition comprising a diacetal and component (B) achieves a synergetic effect so that aldehyde generation is further suppressed in resin pellets and molded products.

Moreover, there was apprehension that a nucleating agent characteristics of diacetals as a clarifier, especially clarity, might deteriorate by using the above diacetal composition. According to the inventors' research, however, it was found that the clarity of the resulting polyolefin resin molded product was not substantially impaired.

The present invention has been accomplished based on these findings and further researches. The present invention provides the following agent for suppressing transfer of odor and taste originating from a diacetal, diacetal composition, polyolefin nucleating agent, polyolefin resin composition and polyolefin resin molded product.

Item 1. An agent for suppressing transfer of odor and taste originating from (A) at least one diacetal represented by the formula (1): 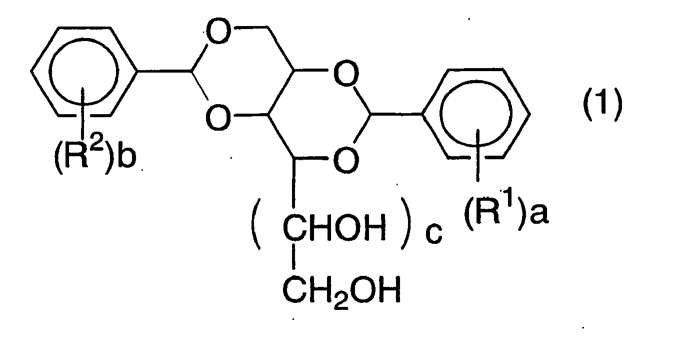 wherein R¹ and R² are the same or different and each represents a hydrogen atom, a C₁ to C₄ alkyl group, a C₁ to C₄ alkoxy group, a C₁ to C₄ alkoxycarbonyl group or a halogen atom; a and b each represents an integer of 1 to 5; c is 0 or 1; when a is 2, the two R¹ groups taken together with the benzene ring to which they are linked may form a tetralin ring; and when b is 2, the two R² groups taken together with the benzene ring to which they are linked may form a tetralin ring; the agent comprising components (B) and (C), wherein component (B) is at least one member selected from the group consisting of:
(B1) C₆ to C₃₂ saturated or unsaturated aliphatic alcohols; and
(B2) C₈ to C₃₂ saturated or unsaturated aliphatic carboxylic acids having at least one hydroxyl group per molecule, and
   component (C) is
(C1) at least one anionic surfactant selected from the group consisting of C₆ to C₃₀ saturated or unsaturated aliphatic alcohol sulfuric ester salts, polyoxyethylene alkyl (C₈ to C₂₂) or alkenyl (C₈ to C₂₂) ether sulfuric ester salts in which the number of moles of ethylene oxide added is 1 to 8, polyoxyethylene alkyl (C₈ to C₂₂) phenyl ether sulfuric ester salts in which the number of moles of ethylene oxide added is 1 to 10, sulfuric ester salts of polyhydric alcohol fatty acid partial esters formed from a C₃ to C₆ polyhydric alcohol and a C₈ to C₂₂ saturated or unsaturated fatty acid, and C₈ to C₂₂ saturated or unsaturated fatty acid monoalkanol (C₂ to C₆) amide sulfuric ester salts, wherein the sulfuric ester salts are lithium salts, sodium salts, potassium salts and ammonium salts;
(C2) at least one member selected from the group consisting of alkali metal salts of C₈ to C₃₂ saturated or unsaturated fatty acids which may have at least one hydroxyl group per molecule;
(C3) at least one aliphatic amine selected from the group consisting of dialkanolamine, trialkanolamine, and di(C₈ to C₂₂ alkyl or alkenyl) methylamine; or
(C4) a mixture of at least two of (C1), (C2) and (C3).

Item 2. The agent for suppressing transfer of odor and taste according to item 1, wherein
component (B) is at least one member selected from the group consisting of 9-hydroxystearic acid, 10-hydroxystearic acid, 12-hydroxystearic acid, 9,10-dihydroxystearic acid, lauryl alcohol, myristyl alcohol, palmityl alcohol, stearyl alcohol and behenyl alcohol, and
component (C) is (C2a) at least one member selected from the group consisting of lithium salts, sodium salts and potassium salts of C₈ to C₃₂ saturated or unsaturated fatty acids which may have at least one hydroxyl group per molecule, or
component (C) is (C1a) at least one sulfuric ester salt selected from the group consisting of lauryl sulfate salts, stearyl sulfate salts, oleyl sulfate salts, polyoxyethylene (the number of moles of ethylene oxide added = 2 to 3) lauryl ether sulfate salts, polyoxyethylene (the number of moles of ethylene oxide added = 2 to 3) stearyl ether sulfate salts, polyoxyethylene (the number of moles of ethylene oxide added = 2 to 3) nonylphenyl ether sulfate salts, polyoxyethylene (the number of moles of ethylene oxide added = 2 to 3) dodecylphenyl ether sulfate salts, glyceryl monolaurate sulfate salts, glyceryl monostearate sulfate salt, lauric acid monoethanolamide sulfuric ester salts, stearic acid monoethanolamide sulfuric ester salts, and oleic acid monoethanolamide sulfuric ester salts, wherein the sulfuric ester salts or sulfate salts are lithium salts, sodium salts and potassium salts.

Item 3. The agent for suppressing transfer of odor and taste according to item 2, wherein component (C) is at least one member selected from the group consisting of sodium lauryl sulfate, potassium lauryl sulfate, sodium stearate, potassium stearate, sodium 12-hydroxystearate and potassium 12-hydroxystearate.

Item 4. The agent for suppressing transfer of odor and taste according to any one of items 1-3, wherein the weight ratio of component (B) to component (C) is 1:0.2 to 5.

Item 5. A method for suppressing transfer of odor and taste originating from (A) at least one diacetal represented by the formula (1): 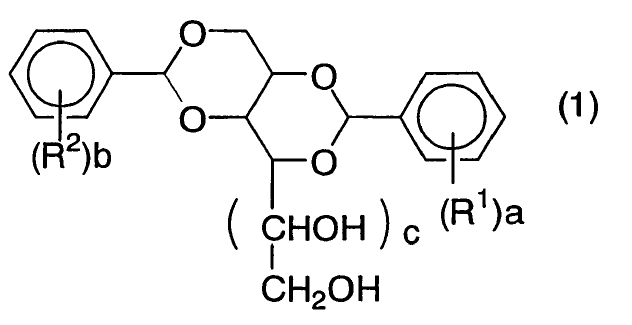 wherein R¹ and R² are the same or different and each represents a hydrogen atom, a C₁ to C₄ alkyl group, a C₁ to C₄ alkoxy group, a C₁ to C₄ alkoxycarbonyl group or a halogen atom; a and b each represents an integer of 1 to 5; c is 0 or 1; when a is 2, the two R¹ groups taken together with the benzene ring to which they are linked may form a tetralin ring; and when b is 2, the two R² groups taken together with the benzene ring to which they are linked may form a tetralin ring
or a method for suppressing aldehyde generation by thermal decomposition of the diacetal;
the method comprising adding the following components (B) and (C) to the diacetal,
wherein component (B) is at least one member selected from the group consisting of:
(B1) C₆ to C₃₂ saturated or unsaturated aliphatic alcohols; and
(B2) C₈ to C₃₂ saturated or unsaturated aliphatic carboxylic acids having at least one hydroxyl group per molecule, and
   component (C) is
(C1) at least one anionic surfactant selected from the group consisting of C₆ to C₃₀ saturated or unsaturated aliphatic alcohol sulfuric ester salts, polyoxyethylene alkyl (C₈ to C₂₂) or alkenyl (C₈ to C₂₂) ether sulfuric ester salts in which the number of moles of ethylene oxide added is 1 to 8, polyoxyethylene alkyl (C₈ to C₂₂) phenyl ether sulfuric ester salts in which the number of moles of ethylene oxide added is 1 to 10, sulfuric ester salts of polyhydric alcohol fatty acid partial esters formed from a C₃ to C₆ polyhydric alcohol and a C₈ to C₂₂ saturated or unsaturated fatty acid, and C₈ to C₂₂ saturated or unsaturated fatty acid monoalkanol (C₂ to C₆) amide sulfuric ester salts, wherein the sulfuric ester salts are lithium salts, sodium salts, potassium salts and ammonium salts;
(C2) at least one member selected from the group consisting of alkali metal salts of C₈ to C₃₂ saturated or unsaturated fatty acids which may have at least one hydroxyl group per molecule;
(C3) at least one aliphatic amine selected from the group consisting of dialkanolamine, trialkanolamine, and di(C₈ to C₂₂ alkyl or alkenyl) methylamine; or
(C4) a mixture of at least two of (C1), (C2) and (C3).

Item 6. The method according to item 5, wherein component (B) is at least one member selected from the group consisting of 9-hydroxystearic acid, 10-hydroxystearic acid, 12-hydroxystearic acid, 9,10-dihydroxystearic acid, lauryl alcohol, myristyl alcohol, palmityl alcohol, stearyl alcohol and behenyl alcohol, and
component (C) is (C2a) at least one member selected from the group consisting of lithium salts, sodium salts and potassium salts of C₈ to C₃₂ saturated or unsaturated fatty acids which may have at least one hydroxyl group per molecule, or
component (C) is (C1a) at least one sulfuric ester salt selected from the group consisting of lauryl sulfate salts, stearyl sulfate salts, oleyl sulfate salts, polyoxyethylene (the number of moles of ethylene oxide added = 2 to 3) lauryl ether sulfate salts, polyoxyethylene (the number of moles of ethylene oxide added = 2 to 3) stearyl ether sulfate salts, polyoxyethylene (the number of moles of ethylene oxide added = 2 to 3) nonylphenyl ether sulfate salts, polyoxyethylene (the number of moles of ethylene oxide added = 2 to 3) dodecylphenyl ether sulfate salts, glyceryl monolaurate sulfate salts, glyceryl monostearate sulfate salts, lauric acid monoethanolamide sulfuric ester salts, stearic acid monoethanolamide sulfuric ester salts, and oleic acid monoethanolamide sulfuric ester salts, wherein the sulfuric ester salts or sulfate salts are lithium salts, sodium salts and potassium salts.

Item 7. The method according to item 6, wherein component (C) is at least one member selected from the group consisting of sodium lauryl sulfate, potassium lauryl sulfate, sodium stearate, potassium stearate, sodium 12-hydroxystearate and potassium 12-hydroxystearate.

Item 8. The method according to any one of items 5-7, wherein the weight ratio of component (B) to component (C) is 1:0.2 to 5.

Item 9. A granular or powdery diacetal composition wherein transfer of odor and taste originating from the diacetal is suppressed;
the composition comprising components (A), (B) and (C), wherein component (A) is at least one diacetal represented by the formula (1) 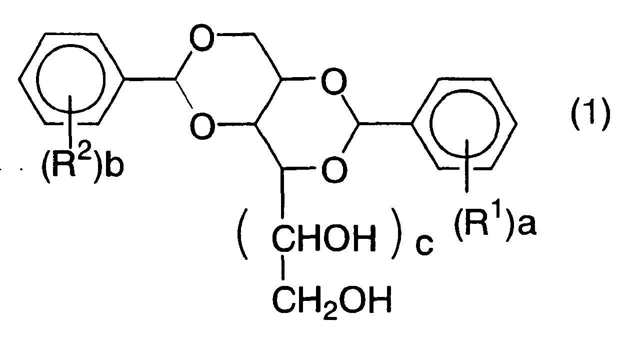 wherein R¹ and R² are the same or different and each represents a hydrogen atom, a C₁ to C₄ alkyl group, a C₁ to C₄ alkoxy group, a C₁ to C₄ alkoxycarbonyl group or a halogen atom; a and b each represents an integer of 1 to 5; c is 0 or 1; when a is 2, the two R¹ groups taken together with the benzene ring to which they are linked may form a tetralin ring; and when b is 2, the two R² groups taken together with the benzene ring to which they are linked may form a tetralin ring, component (B) is at least one member selected from the group consisting of:
(B1) C₆ to C₃₂ saturated or unsaturated aliphatic alcohols; and
(B2) C₈ to C₃₂ saturated or unsaturated aliphatic carboxylic acids having at least one hydroxyl group per molecule, and
   component (C) is
(C1) at least one anionic surfactant selected from the group consisting of C₆ to C₃₀ saturated or unsaturated aliphatic alcohol sulfuric ester salts, polyoxyethylene alkyl (C₈ to C₂₂) or alkenyl (C₈ to C₂₂) ether sulfuric ester salts in which the number of moles of ethylene oxide added is 1 to 8, polyoxyethylene alkyl (C₈ to C₂₂) phenyl ether sulfuric ester salts in which the number of moles of ethylene oxide added is 1 to 10, sulfuric ester salts of polyhydric alcohol fatty acid partial esters formed from a C₃ to C₆ polyhydric alcohol and a C₈ to C₂₂ saturated or unsaturated fatty acid, and C₈ to C₂₂ saturated or unsaturated fatty acid monoalkanol (C₂ to C₆) amide sulfuric ester salts, wherein the sulfuric ester salts are lithium salts, sodium salts, potassium salts and ammonium salts;
(C2) at least one member selected from the group consisting of alkali metal salts of C₈ to C₃₂ saturated or unsaturated fatty acids which may have at least one hydroxyl group per molecule;
(C3) at least one aliphatic amine selected from the group consisting of dialkanolamine, trialkanolamine, and di(C₈ to C₂₂ alkyl or alkenyl) methylamine; or
(C4) a mixture of at least two of (C1), (C2) and (C3).
Item 10. The diacetal composition according to item 9, wherein based on the total amount of components (A), (B) and (C), component (B) is present in a proportion of 0.1 to 5 wt% and component (C) is present in a proportion of 0.1 to 5 wt%.
Item 11. The diacetal composition according to item 10, wherein the weight ratio of component (B) to component (C) is 1:0.2 to 5.
Item 12. A polyolefin resin nucleating agent comprising the diacetal composition according to any one of items 9 to 11, wherein transfer of odor and taste originating from the diacetal is suppressed.
Item 13. A polyolefin resin composition comprising the polyolefin resin nucleating agent according to item 12 and a polyolefin resin, wherein transfer of odor and taste originating from the diacetal is suppressed.
Item 14. The polyolefin resin composition according to item 13, wherein the polyolefin resin nucleating agent according to item 18 is present in an amount of 0.05 to 3 weight parts per 100 weight parts of the polyolefin resin.
Item 15. A polyolefin resin molded product prepared by molding the polyolefin resin composition according to item 13 or 14, wherein transfer of odor and taste originating from the diacetal is suppressed.
Item 16. A container or a packaging material for foods, cosmetics or medicines comprising the polyolefin resin molded product according to item 15, wherein transfer of odor and taste originating from the diacetal is suppressed.
Item 17. A method for suppressing odor originating from a diacetal at the time of molding a polyolefin resin, comprising mixing the nucleating agent according to item 12 with a polyolefin resin and molding a resultant resin composition.
Item 18. A method for suppressing transfer of odor and taste originating from a diacetal to a content (such as foods, cosmetics and medicines), characterized in that it comprises placing the content in a packaging material or a container prepared by mixing the nucleating agent according to item 12 with a polyolefin resin and molding a resultant resin composition.

### DETAILED DESCRIPTION OF THE INVENTION

### Component (A): diacetal

The diacetals of which suppression of odor and taste transfer is contemplated by the present invention are represented by the above formula (1).

In the formula (1), examples of C₁ to C₄ alkoxy groups represented by R¹ and R² include methyl group, ethyl group, propyl group, isopropyl group, butyl group, Examples of C₁ to C₄ alkoxy groups include methoxy group, ethoxy group, propoxy group, isopropoxy group, butoxy group. Examples of C₁ to C₄ alkoxycarbonyl groups include methoxycarbonyl group, ethoxyoarobonyl group, propoxycarbonyl group, isopropoxycarbonyl group, Examples of halogen atoms include fluorine atom, chlorine atom, bromine atom, etc.
a and b are each an integer of 1 to 5, preferably 1, 2 or 3. c is preferably 1. There are no particular restrictions on the position of the substituents represented by R¹ and R², but examples include o-, m- and p-positions when a and b are each 1, and 2,4-, 3,4- and 3,5-positions when a and b are each 2, or 2,4,5- and 3,4,5-positions when a and b are each 3.

All of the diacetals represented by the formula (1) above are known or can be readily prepared by a known process, such as those set forth in Japanese Examined Patent Publication S48-43748 and Japanese Unexamined Patent Publications Nos. S53-5165, S57-185287 and H2-231488.

The following are typical examples of the diacetal represented by the formula (1).

1,3:2,4-O-dibenzylidene-D-sorbitol, 1,3:2,4-bis-O-(o-methylbenzylidene)sorbitol, 1,3:2,4-bis-O-(m-methylbenzylidene)-D-sorbitol, 1,3:2,4-bis-O-(methylbenzylidene)-D-sorbitol, 1,3:2,4-bis-O-(m-isopropylbenzylidene)-D-sorbitol, 1,3:2,4-bis-O-(m-n-propylbenzylidene)-D-sorbitol, 1,3:2,4-bis-O-(m-n-butylbenzylidene)-D-sorbitol, 1,3:2,4-bis-O-(p-methylbenzylidene)-D-sorbitol, 1,3:2,4-bis-O-(p-ethylbenzylidene)-D-sorbitol, 1,3:2,4-bis-O-(p-isopropylbenzylidene)-D-sorbitol, 1,3:2,4-bis-O-(p-n-propylbenzylidene)-D-sorbitol, 1,3:2,4-bis-O-(p-n-butylbenzylidene)-D-sorbitol, 1,3:2,4-bis-O-(2,3-dimethylbenzylidene)-D-sorbitol, 1,3:2,4-bis-O-(2,4-dimethylbenzylidene)-D-sorbitol, 1,3:2,4-bis-O-(2,5-dimethylbenzylidene)-D-sorbitol, 1,3:2,4-bis-O-(3,4-dimethylbenzylidene)-D-sorbitol, 1,3:2,4-bis-O-(3,5-dimethylbenzylidene)-D-sorbitol, 1,3:2,4-bis-O-(2,3-diethylbenzylidene)-D-sorbitol, 1,3:2,4-bis-O-(2,4-diethylbenzylidene)-D-sorbitol, 1,3:2,4-bis-O-(2,5-diethylbenzylidene)-D-sorbitol, 1,3:2,4-bis-O-(3,4-diethylbenzylidene)-D-sorbitol, 1,3:2,4-bis-O-(3,5-diethylbenzylidene)-D-sorbitol, 1,3:2,4-bis-O-(2,4,5-trimethylbenzylidene)-D-sorbitol, 1,3:2,4-bis-O-(3,4,5-trimethylbenzylidene)-D-sorbitol, 1,3:2,4-bis-O-(2,4,5-triethylbenzylidene)-D-sorbitol, 1,3:2,4-bis-O-(3,4,5-triethylbenzylidene)-D-sorbitol, 1,3:2,4-bis-O-(p-methyloxycarbonylbenzylidene)-D-sorbitol, 1,3:2,4-bis-O-(p-ethyloxycarbonylbenzylidene)-D-sorbitol, 1,3:2,4-bis-O-(p-isopropyloxycarbonylbenzylidene)-D-sorbitol, 1,3:2,4-bis-O-(o-n-propyloxycarbonylbenzylidene)-D-sorbitol, 1,3:2,4-bis-O-(o-n-butylbenzylidene)-D-sorbitol, 1,3:2,4-bis-O-(o-chlorobenzylidene)-D-sorbitol, 1,3:2,4-bis-O-(p-chlorobenzylidene)-D-sorbitol, 1,3:2,4-bis-O-[(5,6,7,8-tetrahydro-1-naphthalene)-1-methylene)]-D-sorbitol, 1,3:2,4-bis-O-[(5,6,7,8-tetrahydro-2-naphthalene)-1-methylene]-D-sorbitol, 1,3-O-benzylidene-2,4-O-p-methylbenzylidene-D-sorbitol, 1,3-O-p-methylbenzylidene-2,4-O-benzylidene-D-sorbitol, 1,3-O-benzylidene-2,4-O-p-ethylbenzylidene-D-sorbitol, 1,3-O-p-ethylbenzylidene-2,4-O-benzylidene-D-sorbitol, 1,3-O-benzylidene-2,4-O-p-chlorobenzylidene-D-sorbitol, 1,3-O-p-chlorobenzylidene-2,4-O-benzylidene-D-sorbitol, 1,3-O-benzylidene-2,4-O-(2,4-dimethylbenzylidene)-D-sorbitol, 1,3-O-(2,4-dimethylbenzylidene)-2,4-O-benzylidene-D-sorbitol, 1,3-O-benzylidene-2,4-O-(3,4-dimethylbenzylidene)-D-sorbitol, 1,3-O-(3,4-dimethylbenzylidene)-2,4-O-benzylidene-D-sorbitol, 1,3-O-p-methyl-benzylidene-2,4-O-p-ethylbenzylidene sorbitol, 1,3-p-ethyl-benzylidene-2,4-p-methylbenzylidene-D-sorbitol, 1,3-0-p-methyl-benzylidene-2,4-O-p-chlorobenzylidene-D-sorbitol, and 1,3-O-p-chlorobenzylidene-2,4-O-p-methylbenzylidene-D-sorbitol. These can be used singly or at least two of them may be used as suitably combined.

Among them, preferable are more effective compounds such as 1,3:2,4-O-dibenzylidene-D-sorbitol, 1,3:2,4-bis-O-(o-methylbenzylidene)sorbitol, 1,3:2,4-bis-O-(p-methylbenzylidene)-D-sorbitol, 1,3:2,4-bis-O-(p-ethylbenzylidene)-D-sorbitol, 1,3:2,4-bis-O-(p-isopropylbenzylidene)-D-sorbitol, 1,3:2,4-bis-O-(p-n-propylbenzylidene)-D-sorbitol, 1,3:2,4-bis-O-(p-n-butylbenzylidene)-D-sorbitol, 1,3:2,4-bis-O-(2,4-dimethylbenzylidene)-D-sorbitol, 1,3:2,4-bis-O-(3,4-dimethylbenzylidene)-D-sorbitol, 1,3:2,4-bis-O-(3,5-dimethylbenzylidene)-D-sorbitol, 1,3:2,4-bis-O-(2,4,5-trimethylbenzylidene)-D-sorbitol, 1,3:2,4-bis-O-(p-methyloxycarbonylbenzylidene)-D-sorbitol, 1,3:2,4-bis-O-[(5,6,7,8-tetrahydro-1-naphthalene)-1-methylene)]-D-sorbitol, 1,3:2,4-bis-O-[(5,6,7,8-tetrahydro-2-naphthalene)-1-methylene]-D-sorbitol, 1,3-0-benzylidene-2,4-O-p-methylbenzylidene-D-sorbitol, 1,3-0-p-methylbenzylidene-2,4-O-benzylidene-D-sorbitol, 1,3-O-benzylidene-2,4-O-(2,4-dimethylbenzylidene)-D-sorbitol, 1,3-O-(2,4-dimethylbenzylidene)-2,4-O-benzylidene-D-sorbitol, 1,3-O-benzylidene-2,4-O-(3,4-dimethylbenzylidene)-D-sorbitol, and 1,3-O-(3,4-dimethylbenzylidene)-2,4-O-benzylidene-D-sorbitol. These may be used singly or at least two of them may be used as suitably combined.

Among them, 1,3:2,4-O-dibenzylidene-D-sorbitol, 1,3:2,4-bis-O-(p-methylbenzylidene)-D-sorbitol, 1,3:2,4-bis-O-(p-ethylbenzylidene)-D-sorbitol, and 1,3:2,4-bis-O-(3,4-dimethylbenzylidene)-D-sorbitol are particularly preferable.

There are no particular restrictions on the crystal form of the diacetal as long as the effect of the invention can be achieved, and any crystal form can be used, such as hexagonal, monoclinic, cubic, trigonal and orthorhombic. These crystals are known or can be manufactured by a known method.

The diacetal used in the present invention may be one in which the purity of the 1,3:2,4-compound represented by the formula (1) is 100%, but may also be one containing a small amount of impurities.

### Agent for suppressing transfer of odor and taste

When a nucleating agent comprising the diacetal represented by the formula (1) is added to a polyolefin resin to obtain pellets or resin composition, and a resin molded product prepared from the resin composition is used as a container or a packaging material for foods, cosmetics or other products, an odor is emitted due to an aldehyde released from the diacetal during production process of the resin molded product. In addition, when foods, cosmetics or other products are contained in the container or the packaging material prepared from the molded product, odor and taste will transfer to these products. An object of the present invention is to provide a method for suppressing aldehyde generation by thermal decomposition of the diacetal (method for reducing an amount of aldehyde generation) or to suppress transfer of the odor and taste from the molded product to foods, cosmetics or other products.

To this end, the following or components (B) and (C) are used as an agent for suppressing odor and taste transfer in the present invention.

### <Component (B)>

Component (B) used in the present invention is at least one member selected from the group consisting of: (B1) C₆ to C₃₂ saturated or unsaturated aliphatic alcohols; and (B2) C₈ to C₃₂ saturated or unsaturated aliphatic carboxylic acids having at least one hydroxyl group per molecule.

Examples of saturated or unsaturated aliphatic alcohols (B1) include C₆ to C₃₂, preferably C₁₀ to C₂₂, saturated or unsaturated aliphatic alcohols. Specific examples are hexanol, octanol, lauryl alcohol, myristyl alcohol, palmityl alcohol, stearyl alcohol, isostearyl alcohol, behenyl alcohol and oleyl alcohol. Among them, lauryl alcohol, myristyl alcohol, palmityl alcohol, stearyl alcohol and behenyl alcohol are recommended.

Examples of saturated or unsaturated aliphatic monocarboxylic acids having at least one hydroxyl group per molecule (B2) include C₈ to C₃₂, preferably C₁₂ to C₂₂, aliphatic monocarboxylic acids having at least one, especially one to two, hydroxyl groups per molecule. Specific examples are 9-hydroxystearic acid, 10-hydroxystearic acid, 12-hydroxystearic acid, 9,10-dihydroxystearic acid. Among them, 12-hydroxystearic acid is recommended. These may be used singly or at least two of them may be used as suitably combined.

### <Component (C)>

Examples of component (C) used together with component (B) in the present invention are the following (C1), (C2), (C3) and a mixture thereof (C4).
(C1): at least one anionic surfactant selected from the group consisting of C₆ to C₃₀ saturated or unsaturated aliphatic alcohol sulfuric ester salts, polyoxyethylene alkyl (C₈ to C₂₂) or alkenyl (C₈ to C₂₂) ether sulfuric ester salts in which the number of moles of ethylene oxide added is 1 to 8, polyoxyethylene alkyl (C₈ to C₂₂) phenyl ether sulfuric ester salts in which the number of moles of ethylene oxide added is 1 to 10, sulfuric ester salts of polyhydric alcohol fatty acid partial esters formed from a C₃ to C₆ polyhydric alcohol and a C₈ to C₂₂ saturated or unsaturated fatty acid, and C₈ to C₂₂ saturated or unsaturated fatty acid monoalkanol (C₂ to C₆) amide sulfuric ester salts, wherein the sulfuric ester salts are lithium salts, sodium salts, potassium salts and ammonium salts,
(C2): at least one member selected from the group consisting of alkali metal salts of C₈ to C₃₂ saturated or unsaturated fatty acids which may have at least one hydroxyl group per molecule,
(C3): at least one aliphatic amine selected from the group consisting of dialkanolamine, trialkanolamine, and di(C₈ to C₂₂ alkyl or alkenyl) methylamine, or
(C4): a mixture of at least two of (C1), (C2) and (C3).

Examples of sulfuric ester salt (C1) used in the present invention include C₆ to C₃₀, preferably C₁₀ to C₂₀, saturated or unsaturated aliphatic alcohol sulfuric ester salts; polyoxyethylene alkyl (C₈ to C₂₂, preferably C₁₀ to C₂₂) or alkenyl (C₈ to C₂₂, preferably C₁₀ to C₂₂) ether sulfuric ester salts in which the number of moles of ethylene oxide added is 1 to 8, preferably 2 to 5; polyoxyethylene alkyl (C₈ to C₂₂, preferably C₉ to C₂₀) phenyl ether sulfuric ester salts in which the number of moles of ethylene oxide added is 1 to 10, preferably 2 to 5; sulfuric ester salts of polyhydric alcohol fatty acid partial esters formed from a C₃ to C₆, preferably C₃ or C₄, polyhydric alcohol and a C₈ to C₂₂, preferably C₁₀ to C₂₀, saturated or unsaturated fatty acid; and C₈ to C₂₂, preferably C₁₀ to C₂₀, saturated or unsaturated fatty acid alkanol(C₂ to C₆, preferably C₂ to C₄) amide sulfuric ester salts. The sulfuric ester salt is a lithium salt, sodium salt, potassium salt, or ammonium salt.

Among the above sulfuric ester salts (C1), those represented by the following formulae (a), (b) and (c) are preferred.
- A saturated or unsaturated aliphatic alcohol sulfuric ester salt represented by the formula (a):

   **R^{a} - OSO₃M** (a)

   wherein R^{a} is a C₆ to C₃₀, preferably C₁₀ to C₂₀, saturated or unsaturated aliphatic group (particularly an alkyl or alkenyl group), and M is Li, Na, K or NH₄.
- A polyoxyethylene alkyl or alkenyl ether sulfuric ester salt represented by the formula (b):

   **R^{b}O-(CH₂CH₂O)ₘ-SO₃M** (b)

   wherein R^{b} is an alkyl group (C₈ to C₂₂, preferably C₁₀ to C₂₂) or alkenyl group (C₈ to C₂₂, preferably C₁₀ to C₂₂), m is an integer of 1 to 8, preferably 2 to 5, and M is Li, Na, K or NH₄.
- A polyoxyethylene alkylphenyl ether sulfuric ester salt represented by the formula (c): 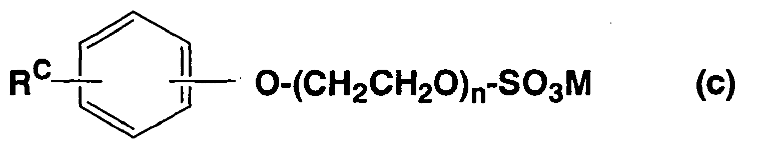 wherein R^{c} is an alkyl group (C₈ to C₂₂, preferably C₉ to C₂₀), n is an integer of 1 to 10, preferably 2 to 5, and M is Li, Na, K or NH₄.

Specific examples of component (C1) include sodium lauryl sulfate, sodium stearyl sulfate, sodium oleyl sulfate, sodium polyoxyethylene (the number of moles of ethylene oxide added = 2 to 3) lauryl ether sulfate, sodium polyoxyethylene (the number of moles of ethylene oxide added = 2 to 3) stearyl ether sulfate, sodium polyoxyethylene (the number of moles of ethylene oxide added = 2 to 3) nonylphenyl ether sulfate, sodium polyoxyethylene (the number of moles of ethylene oxide added = 2 to 3) dodecylphenyl ether sulfate, sodium glyceryl monolaurate sulfate, sodium glyceryl monostearate sulfate, sodium lauric acid monoethanolamide sulfate, sodium stearic acid monoethanolamide sulfate, and sodium oleic acid monoethanolamide sulfate.

Preferred examples include sodium lauryl sulfate, sodium stearyl sulfate, sodium oleyl sulfate, sodium polyoxyethylene (the number of moles of ethylene oxide added = 2 to 3) lauryl ether sulfate, sodium polyoxyethylene (the number of moles of ethylene oxide added = 2 to 3) stearyl ether sulfate, sodium polyoxyethylene (the number of moles of ethylene oxide added = 2 to 3) nonylphenyl ether sulfate, and sodium polyoxyethylene (the number of moles of ethylene oxide added = 2 to 3) dodecylphenyl ether sulfate.

In addition to the above sodium salts, other examples of the above-mentioned sulfuric ester salts include lithium salts, potassium salts, and ammonium salts. The above-mentioned sulfuric ester salts are lithium salts, sodium salts, potassium salts or ammonium salts, among which lithium salts, sodium salts or potassium salts are recommended. The anionic surfactants listed above can be used singly or at least two of them may be used as suitably combined.

Examples of the alkali metal salts of saturated or unsaturated fatty acids (C2) which may have at least one hydroxyl group per molecule include metal salts of C₈ to C₃₂, preferably C₁₀ to C₂₂ saturated or unsaturated fatty acids that may have at least one (particularly one or two, especially one) hydroxyl group per molecule. These can be used singly or at least two of them may be used as suitably combined.

Specific examples are lithium salts, sodium salts, potassium salts, rubidium salts and cesium salts of octanoic acid, capric acid, lauric acid, myristic acid, palmitic acid, stearic acid, 12-hydroxystearic acid, behenic acid, montanic acid, oleic acid, linoleic acid, linolenic acid, eleostearic acid, ricinoleic acid and erucic acid. Among them, lithium salts, sodium salts and potassium salts of lauric acid, myristic acid, palmitic acid, stearic acid, and 12-hydroxystearic acid are particularly preferred.

Examples of the aliphatic amine (C3) used in the invention include di- or tri- alkanolamines, particularly di- or tri-(C₁ to C₄ alkanol)amines such as diethanolamine, dipropanolamine, diisopropanolamine, butanol amine, triethanolamine, tripropanolamine, triisopropanolamine and tributanolamine and di(C₈ to C₂₂ alkyl or alkenyl)methylamines such as distearylmethylamine, dihexadecylmethylamine, ditetradeoylmethylamine, didodecylmethylamine, dioleoylmethylamine and stearyllaurylmethylamine. Among them, diethanolamine and diisopropanolamine are preferred. These can be used singly or at least two of them may be used as suitably combined.

It is more preferable that component (C) is at least one member selected from the group consisting of sodium lauryl sulfate, potassium lauryl sulfate, sodium stearate, potassium stearate, sodium 12-hydroxystearate and potassium 12-hydroxystearate.

In the present invention, singly or a combination of components (B) and (C) is used as an agent for suppressing transfer of odor and taste originating from a diacetal as component (A).

When components (B) and (C) are jointly used, the amount of component (B) is in the range of 0.1 to 5 wt%, preferably 1 to 3 wt%, and the amount of component (C) is in the range of 0.1 to 5 wt%, preferably 1 to 3 wt%, based on the total amount of a diacetal as component (A), component (B) and component (C). If the amounts of both components (B) and (C) are less than 0.1 wt%, the effect of suppressing aldehyde generation is so weak that odor and taste evaluations show a tendency to be adversely affected. On the other hand, if the amounts of both components (B) and (C) are more than 5 wt%, nucleating agent characteristics as a clarifier of polyolefin resin tend to deteriorate.
The weight ratio of component (B) to component (C) is in the range of 1:0.2 to 5, preferably 1:0.5 to 3. Within this range, the combined use of components (B) and (C) tends to be remarkably effective.

### Diacetal composition of the present invention

The diacetal composition of the present invention comprises said amount of the agent for suppressing odor and taste, with the balance being diacetal (A) represented by the formula (1).

Specifically, the diacetal composition is a powdery or granular diacetal composition comprising:
ii) diacetal (A) represented by the formula (1), at least one member (component (B)) selected from the group consisting of (B1) saturated or unsaturated aliphatic alcohols and (B2) saturated or unsaturated aliphatic carboxylic acids having at least one hydroxyl group per molecule, and at least one member (component (C)) selected from the group consisting of (C1) anionic surfactants, (C2) alkali metal salts of fatty acids and (C3) aliphatic amines.

The diacetal composition according to the present invention is composed of components (A), (B) and (C), and it is preferable that the amount of component (B) is in the range of 0.1 to 5 wt%, preferably 1 to 3 wt%, and the amount of component (C) is in the range of 0.1 to 5 wt%, preferably 1 to 3 wt% based on the diacetal composition, with the balance being diacetal (A) represented by the formula (1). If the amounts of both components (B) and (C) are less than 0.1 wt%, the effect of suppressing aldehyde generation is so weak that odor and taste evaluations show a tendency to be adversely affected. On the other hand, if the amounts of both components (B) and (C) are more than 5 wt%, nucleating agent characteristics as a clarifier of polyolefin resins tend to deteriorate.

The weight ratio of component (B) to component (C) is in the range of 1:0.2 to 5, preferably 1:0.5 to 3. Within this range, the combined use of components (B) and (C) tends to be remarkably effective.

The diacetal composition according to the present invention is not particularly limited and suitably selected. Typical preferred examples are a combination of the following components (A), (B) and (C):
component (A) : at least one member selected from the group consisting of 1,3:2,4-O-dibenzylidene-D-sorbitol, 1,3:2,4-bis-O-(p-methylbenzylidene)-D-sorbitol, 1,3:2,4-bis-O-(p-ethylbenzylidene)-D-sorbitol, and 1,3:2,4-bis-O-(3,4-dimethylbenzylidene)-D-sorbitol,
component (B) : at least one member selected from the group consisting of 9-hydroxystearic acid, 10-hydroxystearic acid, 12-hydroxystearic acid, 9,10-dihydroxystearic acid, lauryl alcohol, myristyl alcohol, palmityl alcohol, stearyl alcohol and behenyl alcohol,
component (C) : at least one member selected from the group consisting of lithium lauryl sulfate, sodium lauryl sulfate, potassium lauryl sulfate, lithium polyoxyethylene (the number of moles of ethylene oxide added = 2 to 3) lauryl sulfate, sodium polyoxyethylene (the number of moles of ethylene oxide added = 2 to 3) lauryl sulfate, potassium polyoxyethylene (the number of moles of ethylene oxide added = 2 to 3) lauryl sulfate, sodium laurate, potassium laurate, sodium myristate, potassium myristate, sodium palmitate, potassium palmitate, sodium stearate, potassium stearate, sodium behenate, sodium montanate, sodium 12-hydroxystearate, potassium 12-hydroxystearate, diethanolamine and diisopropanolamine.

Preferred examples of the diacetal composition comprising (A) and (B) include the following:
1,3:2,4-O-dibenzylidene-D-sorbitol + lauryl alcohol,
1,3:2,4-O-dibenzylidene-D-sorbitol + myristyl alcohol,
1,3:2,4-O-dibenzylidene-D-sorbitol + palmityl alcohol,
1,3:2,4-O-dibenzylidene-D-sorbitol + stearyl alcohol,
1,3:2,4-O-dibenzylidene-D-sorbitol + 12-hydroxystearic acid,
1,3:2,4-bis-O-(p-methylbenzylidene)-D-sorbitol + lauryl alcohol,
1,3:2,4-bis-O-(p-methylbenzylidene)-D-sorbitol + myristyl alcohol,
1,3:2,4-bis-O-(p-methylbenzylidene)-D-sorbitol + palmityl alcohol,
1,3:2,4-bis-O-(p-methylbenzylidene)-D-sorbitol + stearyl alcohol,
1,3:2,4-bis-O-(p-methylbenzylidene)-D-sorbitol + 12-hydroxystearic acid,
1,3:2,4-bis-O-(p-ethylbenzylidene)-D-sorbitol + lauryl alcohol,
1,3:2,4-bis-O-(p-ethylbenzylidene)-D-sorbitol + myristyl alcohol,
1,3:2,4-bis-O-(p-ethylbenzylidene)-D-sorbitol + palmityl alcohol,
1,3:2,4-bis-O-(p-ethylbenzylidene)-D-sorbitol + stearyl alcohol,
1,3:2,4-bis-O-(p-ethylbenzylidene)-D-sorbitol + 12-hydroxystearic acid,
1,3:2,4-bis-O-(3,4-dimethylbenzylidene)-D-sorbitol + lauryl alcohol,
1,3:2,4-bis-O-(3,4-dimethylbenzylidene)-D-sorbitol + myristyl alcohol,
1,3:2,4-bis-O-(3,4-dimethylbenzylidene)-D-sorbitol + palmityl alcohol,
1,3:2,4-bis-O-(3,4-dimethylbenzylidene)-D-sorbitol + stearyl alcohol and
1,3:2,4-bis-O-(3,4-dimethylbenzylidene)-D-sorbitol + 12-hydroxysteario acid.

Preferred examples of the diacetal composition comprising components (A), (B) and (C) include the following:
(1) 1,3:2,4-O-dibenzylidene-D-sorbitol + lauryl alcohol + sodium stearate,
   1,3:2,4-O-dibenzylidene-D-sorbitol + myristyl alcohol + sodium stearate,
   1,3:2,4-O-dibenzylidene-D-sorbitol + palmityl alcohol + sodium stearate,
   1,3:2,4-O-dibenzylidene-D-sorbitol + stearyl alcohol + sodium stearate,
   1,3:2,4-O-dibenzylidene-D-sorbitol + 12-hydroxystearic acid + sodium laurate,
   1,3:2,4-O-dibenzylidene-D-sorbitol + 12-hydroxystearic acid + sodium palmitate,
   1,3:2,4-O-dibenzylidene-D-sorbitol + 12-hydroxystearic acid + lithium stearate,
   1,3:2,4-O-dibenzylidene-D-sorbitol + 12-hydroxystearic acid + potassium stearate,
   1,3:2,4-O-dibenzylidene-D-sorbitol + 12-hydroxystearic acid + sodium 12-hydroxystearate,
   1,3:2,4-O-dibenzylidene-D-sorbitol + 12-hydroxystearic acid + sodium stearate,
   1,3:2,4-O-dibenzylidene-D-sorbitol + 12-hydroxystearic acid + potassium 12-hydroxystearate,
   1,3:2,4-O-dibenzylidene-D-sorbitol + 12-hydroxystearic acid + sodium behenate,
   1,3:2,4-O-dibenzylidene-D-sorbitol + 12-hydroxystearic acid + sodium montanate,
   1,3:2,4-O-dibenzylidene-D-sorbitol + 12-hydroxystearic acid + sodium oleate,
   1,3:2,4-O-dibenzylidene-D-sorbitol + 12-hydroxystearic acid + lithium lauryl sulfate,
   1,3:2,4-O-dibenzylidene-D-sorbitol + 12-hydroxystearic acid + sodium lauryl sulfate,
   1,3:2,4-O-dibenzylidene-D-sorbitol + 12-hydroxystearic acid + potassium lauryl sulfate,
   1,3:2,4-O-dibenzylidene-D-sorbitol + 12-hydroxystearic acid + potassium oleyl sulfate,
   1,3:2,4-O-dibenzylidene-D-sorbitol + 12-hydroxystearic acid + sodium polyoxyethylene (3 moles added) lauryl sulfate,
   1,3:2,4-O-dibenzylidene-D-sorbitol + 12-hydroxystearic acid + sodium glyceryl monolaurate sulfate,
   1,3:2,4-O-dibenzylidene-D-sorbitol + 12-hydroxystearic acid + diethanolamine,
   1,3:2,4-O-dibenzylidene-D-sorbitol + 12-hydroxystearic acid + triisopropanolamine.
(2) 1,3:2,4-bis-O-(p-methylbenzylidene)-D-sorbitol + lauryl alcohol + sodium stearate,
   1,3:2,4-bis-O-(p-methylbenzylidene)-D-sorbitol + myristyl alcohol + sodium stearate,
   1,3:2,4-bis-O-(p-methylbenzylidene)-D-sorbitol + palmityl alcohol + sodium stearate,
   1,3:2,4-bis-O-(p-methylbenzylidene)-D-sorbitol + stearyl alcohol + sodium stearate,
   1,3:2,4-bis-O-(p-methylbenzylidene)-D-sorbitol + 12-hydroxystearic acid + sodium laurate,
   1,3:2,4-bis-O-(p-methylbenzylidene)-D-sorbitol + 12-hydroxystearic acid + sodium palmitate,
   1,3:2,4-bis-O-(p-methylbenzylidene)-D-sorbitol + 12-hydroxystearic acid + lithium stearate,
   1,3:2,4-bis-O-(p-methylbenzylidene)-D-sorbitol + 12-hydroxystearic acid + sodium stearate,
   1,3:2,4-bis-O-(p-methylbenzylidene)-D-sorbitol + 12-hydroxystearic acid + potassium 12-hydroxystearate,
   1,3:2,4-bis-O-(p-methylbenzylidene)-D-sorbitol + 12-hydroxysteario acid + sodium behenate,
   1,3:2,4-bis-O-(p-methylbenzylidene)-D-sorbitol + 12-hydroxystearic acid + sodium montanate,
   1,3:2,4-bis-O-(p-methylbenzylidene)-D-sorbitol + 12-hydroxystearic acid + sodium oleate,
   1,3:2,4-bis-O-(p-methylbenzylidene)-D-sorbitol + 12-hydroxystearic acid + lithium lauryl sulfate,
   1,3:2,4-bis-O-(p-methylbenzylidene)-D-sorbitol + 12-hydroxystearic acid + sodium lauryl sulfate,
   1,3:2,4-bis-O-(p-methylbenzylidene)-D-sorbitol + 12-hydroxystearic acid + potassium lauryl sulfate,
   1,3:2,4-bis-O-(p-methylbenzylidene)-D-sorbitol + 12-hydroxystearic acid + potassium oleyl sulfate,
   1,3:2,4-bis-O-(p-methylbenzylidene)-D-sorbitol + 12-hydroxystearic acid + potassium stearate,
   1,3:2,4-bis-O-(p-methylbenzylidene)-D-sorbitol + 12-hydroxystearic acid + sodium 12-hydroxystearate,
   1,3:2,4-bis-O-(p-methylbenzylidene)-D-sorbitol + 12-hydroxystearic acid + sodium polyoxyethylene (3 moles added) lauryl sulfate,
   1,3:2,4-bis-o-(p-methylbenzylidene)-D-sorbitol + 12-hydroxystearic acid + sodium glyceryl monolaurate sulfate,
   1,3:2,4-bis-O-(p-methylbenzylidene)-D-sorbitol + 12-hydroxystearic acid + diethanolamine, and
   1,3:2,4-bis-O-(p-methylbenzylidene)-D-sorbitol + 12-hydroxystearic acid + triisopropanolamine.
(3) 1,3:2,4-bis-O-(p-ethylbenzylidene)-D-sorbitol + lauryl alcohol + sodium stearate,
   1,3:2,4-bis-O-(p-ethylbenzylidene)-D-sorbitol + myristyl alcohol + sodium stearate,
   1,3:2,4-bis-O-(p-ethylbenzylidene)-D-sorbitol + palmityl alcohol + sodium stearate,
   1,3:2,4-bis-O-(p-ethylbenzylidene)-D-sorbitol + stearyl alcohol + sodium stearate,
   1,3:2,4-bis-O-(p-ethylbenzylidene)-D-sorbitol + 12-hydroxystearic acid + sodium laurate,
   1,3:2,4-bis-O-(p-ethylbenzylidene)-D-sorbitol + 12-hydroxystearic acid + sodium palmitate,
   1,3:2,4-bis-O-(p-ethylbenzylidene)-D-sorbitol + 12-hydroxysteario acid + lithium stearate,
   1,3:2,4-bis-O-(p-ethylbenzylidene)-D-sorbitol + 12-hydroxystearic acid + sodium stearate,
   1,3:2,4-bis-O-(p-ethylbenzylidene)-D-sorbitol + 12-hydroxystearic acid + potassium 12-hydroxystearate,
   1,3:2,4-bis-O-(p-ethylbenzylidene)-D-sorbitol + 12-hydroxystearic acid + sodium behenate,
   1,3:2,4-bis-O-(p-ethylbenzylidene)-D-sorbitol + 12-hydroxystearic acid + potassium stearate,
   1,3:2,4-bis-O-(p-ethylbenzylidene)-D-sorbitol + 12-hydroxystearic acid + sodium 12-hydroxystearate,
   1,3:2,4-bis-O-(p-ethylbenzylidene)-D-sorbitol + 12-hydroxystearic acid + sodium montanate,
   1,3:2,4-bis-O-(p-ethylbenzylidene)-D-sorbitol + 12-hydroxystearic acid + sodium oleate,
   1,3:2,4-bis-O-(p-ethylbenzylidene)-D-sorbitol + 12-hydroxystearic acid + lithium lauryl sulfate,
   1,3:2,4-bis-O-(p-ethylbenzylidene)-D-sorbitol + 12-hydroxystearic acid + sodium lauryl sulfate,
   1,3:2,4-bis-O-(p-ethylbenzylidene)-D-sorbitol + 12-hydroxystearic acid + potassium lauryl sulfate,
   1,3:2,4-bis-O-(p-ethylbenzylidene)-D-sorbitol + 12-hydroxystearic acid + potassium oleyl sulfate,
   1,3:2,4-bis-O-(p-ethylbenzylidene)-D-sorbitol + 12-hydroxystearic acid + sodium polyoxyethylene (3 moles added) lauryl sulfate,
   1,3:2,4-bis-O-(p-ethylbenzylidene)-D-sorbitol + 12-hydroxystearic acid + sodium glyceryl monolaurate sulfate,
   1,3:2,4-bis-O-(p-ethylbenzylidene)-D-sorbitol + 12-hydroxystearic acid + diethanolamine,
   1,3:2,4-bis-O-(p-ethylbenzylidene)-D-sorbitol + 12-hydroxystearic acid + triisopropanolamine.
(4) 1,3:2,4-bis-O-(3,4-dimethylbenzylidene)-D-sorbitol + lauryl alcohol + sodium stearate,
   1,3:2,4-bis-O-(3,4-dimethylbenzylidene)-D-sorbitol + myristyl alcohol + sodium stearate,
   1,3:2,4-bis-O-(3,4-dimethylbenzylidene)-D-sorbitol + palmityl alcohol + sodium stearate,
   1,3:2,4-bis-O-(3,4-dimethylbenzylidene)-D-sorbitol + stearyl alcohol + sodium stearate,
   1,3:2,4-bis-O-(3,4-dimethylbenzylidene)-D-sorbitol + 12-hydroxystearic acid + sodium laurate,
   1,3:2,4-bis-O-(3,4-dimethylbenzylidene)-D-sorbitol + 12-hydroxystearic acid + sodium palmitate,
   1,3:2,4-bis-O-(3,4-dimethylbenzylidene)-D-sorbitol + 12-hydroxystearic acid + lithium stearate,
   1,3:2,4-bis-O-(3,4-dimethylbenzylidene)-D-sorbitol + 12-hydroxystearic acid + sodium stearate,
   1,3:2,4-bis-O-(3,4-dimethylbenzylidene)-D-sorbitol + 12-hydroxystearic acid + potassium 12-hydroxystearate,
   1,3:2,4-bis-O-(3,4-dimethylbenzylidene)-D-sorbitol + 12-hydroxystearic acid + potassium stearate,
   1,3:2,4-bis-O-(3,4-dimethylbenzylidene)-D-sorbitol + 12-hydroxystearic acid + sodium 12-hydroxystearate,
   1,3:2,4-bis-O-(3,4-dimethylbenzylidene)-D-sorbitol + 12-hydroxystearic acid + sodium behenate,
   1,3:2,4-bis-O-(3,4-dimethylbenzylidene)-D-sorbitol + 12-hydroxystearic acid + sodium montanate,
   1,3:2,4-bis-O-(3,4-dimethylbenzylidene)-D-sorbitol + 12-hydroxystearic acid + sodium oleate,
   1,3:2,4-bis-O-(3,4-dimethylbenzylidene)-D-sorbitol + 12-hydroxystearic acid + lithium lauryl sulfate,
   1,3:2,4-bis-O-(3,4-dimethylbenzylidene)-D-sorbitol + 12-hydroxystearic acid + sodium lauryl sulfate,
   1,3:2,4-bis-O-(3,4-dimethylbenzylidene)-D-sorbitol + 12-hydroxystearic acid + potassium lauryl sulfate,
   1,3:2,4-bis-O-(3,4-dimethylbenzylidene)-D-sorbitol + 12-hydroxystearic acid + potassium oleyl sulfate,
   1,3:2,4-bis-O-(3,4-dimethylbenzylidene)-D-sorbitol + 12-hydroxystearic acid + sodium polyoxyethylene (3 moles added) lauryl sulfate,
   1,3:2,4-bis-O-(3,4-dimethylbenzylidene)-D-sorbitol + 12-hydroxystearic acid + sodium glyceryl monolaurate sulfate,
   1,3:2,4-bis-O-(3,4-dimethylbenzylidene)-D-sorbitol + 12-hydroxystearic acid + diethanolamine and
   1,3:2,4-bis-O-(3,4-dimethylbenzylidene)-D-sorbitol + 12-hydroxystearic acid + triisopropanolamine.

The diacetal composition according to the present invention can be easily prepared by adding components (B) and (C) to the diacetals represented by the formula (I).

The method of adding the components is not particularly limited insofar as a desired diacetal composition can be obtained. Examples of such method include: i) addition in the course of manufacturing a diacetal represented by the formula (1) from sorbitol and a corresponding benzaldehyde according to various known processes (such as those set forth in Japanese Unexamined Patent Publication No.1990-231488); ii) powder mixing with a diacetal represented by the formula (1) by means of a mixer such as Henschel mixer, V-blender or ribbon blender; and iii) addition and mixing of the above specified amount components (B) and (C) as such or as dissolved in a solvent such as methanol ethanol having 1-3 carbon atoms and water to a slurry of a diacetal represented by the formula (1) in a dispersion medium such as e.g. methanol ethanol having 1-3 carbon atoms and water, and subsequent removal of the solvent by evaporation.

In the above process i), it is preferable that the specified amount components (B) and (C) is added after a diacetal represented by the formula (1) is formed by diacetalization reaction of a sorbitol and a benzaldehyde.

In the above process iii), the diacetal concentration in the slurry is not particularly limited and in general it is preferably 10 to 60 wt%. The slurry temperature is suitably selected from a wide range and in general it is preferably 20 to 100°C.

The thus obtained diacetal composition containing components (B) and (C) is then pulverized, crushed, granulated or classified, if necessary.

The diacetal composition of the present invention may take any form as suitably selected, and may be in the common form of powders or grains, or in a granulated form such as granules, cylinders and pellets.

In the case of powders, the average particle diameter thereof is 3 to 2000 µm and is preferably 7 to 200 µm. If the average particle diameter is smaller than 3 pm, powder characteristics tends to be poor, and a special pulverization apparatus is necessary.

In the case of granules, a granular diacetal composition of the desired shape and size can be obtained from a powdery diacetal composition obtained by the above method. This granular diacetal composition is advantageous in that it has reduced dust generation and improved particle fluidity, as compared with a powdery diacetal composition.

Any of the above forms can be manufactured using a known granulator, pulverizer/crusher or classifier. Examples of granulators include dry or wet extrusion granulators, mixing and stirring granulators, tableting machines, dry compression roll granulators, and oscillating granulator. Examples of pulverizer/crushers include pin mills, jet mills, pulverizers, cutter mills, hammer mills, planar crushers, flake crushers, nibblers, Examples of classifiers include vibrating sifters, air classifiers.

In the diacetal composition thus obtained, transfer of odor and taste originating from the diacetal represented by the formula (1) is suppressed.

Accordingly, the present invention provides a method for suppressing transfer of odor and taste originating from the diacetal or a method for suppressing aldehyde generation by thermal decomposition of the diacetal, comprising adding a combination of components (B) and (C) to the diacetal represented by the formula (1).

Furthermore, the present invention also provides use of a combination of components (B) and (C) for suppressing transfer of odor and taste originating from the diacetal or for suppressing aldehyde generation by thermal decomposition of the diacetal.

### Polyolefin resin nucleating agent

The polyolefin resin nucleating agent of the present invention may be the present diacetal composition itself or may be prepared by adding a polyolefin resin additive to the diacetal composition.

The diacetal composition used for the present polyolefin resin nucleating agent is useful since it shows extremely low aldehyde generation due to thermal hysteresis and very little decrease in nucleating agent performances. If the present nucleating agent includes an antioxidant, its storage stability can be remarkably improved. Such nucleating agent having good storage stability comprises an antioxidant in an amount of 0.01 to 5 weight parts, preferably 0.01 to 3 weight parts, per 100 weight parts of the diacetal composition.

Examples of such antioxidants include phenol-containing antioxidants, phosphite-containing compounds and sulfur-containing antioxidants. More specifically, 2,6-di-tert-butylphenol, n-octadecyl-3-(3',5'-di-tert-butyl-4'-hydroxyphenyl)propionate, tetrakis[methylene-3-(3,5-di-tert-butyl-4-hydroxyphenyl)propionate]methane, tris(3,5-di-tert-butyl-4-hydroxybenzyl)isooyanurate, 4,4'-butylidene-bis(3-methyl-6-tert-butylphenol) and triethyleneglycol-bis-[3-(3-tert-butyl-4-hydroxy-5-methylphenyl)propionate], are recommended. Among them, tetrakis[methylene-3-(3,5-di-tert-butyl-4-hydroxyphenyl)propionate]methane is especially recommended.

According to purpose or application, a conventional polyolefin modifier can be suitably added to the polyolefin resin nucleating agent of the present invention, insofar as the effect of the present invention is not compromised.

Examples of such polyolefin modifiers include the various additives listed in "The Tables of Positive Lists of Additives" edited by the Japan Hygienic Olefin and Styrene Plastic Association (January, 2002). More specific examples include stabilizers (such as metal compounds, epoxy compounds, nitrogen compounds, phosphorus compounds, and sulfur compounds), UV absorbers (such as benzophenone compounds and benzotriazole compounds), surfactants, lubricants (such as paraffin, wax, and other aliphatic hydrocarbons, C₈ to C₂₂ higher fatty acids, C₈ to C₂₂ higher fatty acid metal (Al, Ca, Mg, Zn) salts, C₈ to C₂₂ higher aliphatic alcohols, polyglycols, esters of C₄ to C₂₂ higher fatty acids and C₄ to C₁₈ aliphatic monohydric alcohols, C₈ to C₂₂ higher fatty acid amides, silicone oils, and rosin derivatives), fillers (such as talc, hydrotalcite, mica, zeolite, perlite, diatomaceous earth, calcium carbonate, and glass fibers), foaming agents, foaming auxiliaries, polymer additives, plasticizers (such as dialkyl phthalates and dialkyl hexahydrophthalates), crosslinking agents, crosslinking accelerators, antistatic agents, flame retardants, dispersants, organic and inorganic pigments, working auxiliaries and other nucleating agents.

Various methods can be employed to mix these additional components. For example, it is preferable to dry blend the diacetal composition of the present invention with the additional components to obtain a uniform mixture.

### Polyolefin resin composition

The polyolefin resin composition according to the present invention is obtained by incorporating the polyolefin resin nucleating agent of the present invention into a polyolefin resin by a standard method.

The polyolefin resin composition of the present invention can be manufactured by any conventional method, with no particular restrictions thereon, as long as the desired resin composition is obtained. For example, a polyolefin resin (powder or flakes), the polyolefin resin nucleating agent of the present invention, and, if needed, a polyolefin modifier discussed below are mixed in a conventional mixer, such as Henschel mixer, V-blender or ribbon blender, to obtain a blend type of polyolefin resin composition. Other examples include a method in which this blend type of polyolefin resin composition is melt kneaded at the desired temperature in a conventional kneader, such as a single screw or twin screw extruder, the extruded strands are cooled, and the strands thus obtained are cut into pellets, as well as a method that is a variation of this pellet type, in which master batch pellets are made from a polyolefin resin nucleating agent and a polyolefin resin.

There are no particular restrictions on the amount of the polyolefin resin nucleating agent according to the present invention to be added to a polyolefin resin, as long as the desired effect is obtained, and this amount can be suitably selected from a wide range. Usually, the amount is 0.05 to 3 weight parts, preferably 0.07 to 1 weight parts, per 100 weight parts of the polyolefin resin. Blending within this range can fully produce the desired effect of the present invention.

A method for adding the diacetal composition to a polyolefin resin may preferably be a direct addition using a conventional machine such as a single screw or twin screw extruder, but is not limited thereto. Alternatively, addition in the form of high concentration master batch of, for example, 2 to 15 wt% is also allowable.

Examples of the polyolefin resin according to the present invention include polyethylene-based resins, polypropylene-based resins, polybutene-based resins, polymethylpentene-based resins, and polybutadiene-based resins. Specific examples are high-density polyethylene, medium-density polyethylene, linear polyethylene, ethylene copolymers with an ethylene content of at least 50 wt% and preferably 70 wt% or higher, propylene homopolymers, propylene copolymers with a propylene content of at least 50 wt% and preferably 70 wt% or higher, butene homopolymers, butene copolymers with a butene content of at least 50 wt% and preferably 70 wt% or higher, methylpentene homopolymers, methylpentene copolymers with a methylpentene content of at least 50 wt% and preferably 70 wt% or higher, and polybutadiene.

The above copolymers may be random copolymers or block copolymers. The stereoregularity of these resins may be either isotactic or syndiotactic.

Specific examples of comonomers which can form the various copolymers above include ethylene, propylene, butene, pentene, hexene, heptene, octene, nonene, decene, undecene dodecene; 1,4-endomethylenecyclohexene; methyl (meth)acrylate, ethyl (meth)acrylat; vinyl acetate.

Catalysts that can be used in the manufacture of these polymers include not only Ziegler-Natta catalysts which are commonly used, but also a catalyst system comprising a combination of a transition metal compound (e.g., a titanium halide such as titanium trichloride or titanium tetrachloride) supported on a support comprising as a main component magnesium chloride, with an alkyl aluminum compound (such as triethyl aluminum or diethyl aluminum chloride), and metallocene catalysts.

The recommended melt flow rate (hereinafter referred to as "MFR", measured according to JIS K 7210-1976) of the polyolefin resin according to the present invention can be suitably selected according to the molding method to be employed, and is usually 0.01 to 200 g/10 minutes, preferably 0.05 to 100 g/10 minutes.

To the extent that the effect of the present invention is not lost, the above-mentioned known polyolefin modifiers can be added to the resin composition of the present invention as dictated by the intended use and application thereof.

Thus obtained polyolefin resin composition according to the present invention is novel and useful having superior nucleating agent performances such as clarity, in which aldehyde generation during the molding process as well as transfer of aldehyde odor and taste in the final molded product are suppressed.

Accordingly, in a polyolefin resin composition comprising a diacetal represented by the formula (1), the present invention also provides a method for suppressing odor originating from the diacetal or a method for suppressing aldehyde generation by thermal decomposition of the diacetal, which comprises adding the polyolefin resin nucleating agent of the invention to a polyolefin resin.

In addition, in a polyolefin resin composition comprising a diacetal represented by the formula (1), the present invention also provides use of the polyolefin resin nucleating agent of the invention for suppressing transfer of odor and taste originating from the diacetal or for suppressing aldehyde generation by thermal decomposition of the diacetal.

### Polyolefin resin moldings

The polyolefin resin moldings of the present invention are obtained by molding the polyolefin resin composition of the present invention according to a conventional molding method. Any known method, such as injection molding, extrusion molding, blow molding, pressure forming, rotational molding, or film forming, can be employed in molding the polyolefin resin composition according to the present invention. The processing conditions can be suitably selected from the wide range of conditions that have been employed in the past.

The polyolefin resin composition of the present invention can be put into use and then molded in the same fields in which polyolefin resin compositions containing a DBS as a nucleating agent have been conventionally used.

In the polyolefin resin molded product according to the present invention, a generation of aldehyde and the like during molding process is suppressed and therefore odor generation is decreased, compared with a molded product prepared from the conventional resin composition containing DBS.

Accordingly, the present invention provides a method for suppressing odor due to aldehyde derived from diacetal at the time of molding a polyolefin resin, comprising mixing the present nucleating agent with the polyolefin resin and molding a resultant resin composition.

In addition, since the amount of aldehyde, is significantly reduced in the molded product of the invention, transfer of odor and taste due to aldehyde is also decreased. Furthermore, the polyolefin resin molded product of the present invention shows superior clarity.

The present invention provides a method for suppressing transfer of odor and taste originating from a diacetal to foods, cosmetics and medicines, **characterized in that** said method comprises placing the content in a packaging material or a container prepared by mixing the nucleating agent of the present invention with a polyolefin resin and molding the resultant resin composition.

Thus the polyolefin resin molded product of the invention can be used advantageously particularly in the fields of packaging materials for foods, containers for foods, cosmetics and medicines. Of course, it is also used in other fields. Examples include medical instruments sterilized by heat or radiation, such as disposable syringes, infusion and transfusion sets, equipment for collecting blood; packaging materials for foods, plants, etc. sterilized by radiation or the like; cases such as cases for clothes, containers for clothes; cups for heat-packaging foods, packaging containers for retort-processed foods; containers for use in microwave oven, containers for cans, vessels; for beverages such as juice, tea; cosmetics, medicines, shampoo; containers and caps for seasonings such as miso, soy sauce; cases and containers for foods such as water, rice, bread, pickles; sundries such as cases for use in refrigerators; stationery; electric and mechanical parts; automobile parts.

### EXAMPLES

The present invention will now be described in detail with reference to examples and comparative examples.

### COMPARATIVE EXAMPLES 1-5

1,3:2,4-di(p-methylbenzylidene)sorbitol (hereinafter referred to as "Me-DBS") and an alcohol or 12-hydroxystaric acid shown in Table 1 were stirred for one hour in methanol (weighing as 6 times as Me-DBS) under reflux to give a mixture in a white paste form. Then methanol was removed under reduced pressure, dried in a vacuum drier for one hour under the pressure of 133 Pa at a temperature of 80°C, to obtain a powdery sample (hereinafter referred to as "Me-DBS composition").

To 100 weight parts of an isotactic random polypropylene resin with an ethylene content of 3.0 wt% (MFR = 20 g/10 minutes; hereinafter referred to as "r-PP"), were added 0.2 weight part of the Me-DBS composition, 0.05 weight part of tetrakis[methylene-3-(3,5-di-tert-butyl-4-hydroxyphenyl)propionate]methane (trade name "Irganox 1010", made by Ciba Specialty Chemicals) and 0.05 weight part of calcium stearate, and these components were blended in a Henschel mixer. Then the mixture was melt kneaded using a single screw extruder having a diameter of 25 mm at a resin temperature of 240°C and pelletized. The odor evaluation of the obtained pellets was carried out by the following dry method. The results are shown in Table 1.

### Odor evaluation of pellets by dry method

A 225 ml glass bottle in which 60 g of pellets were sealed was left to stand in a constant temperature bath of 80°C for 2 hours, and cooled to room temperature. Immediately after that, the odor intensity was rated by ten panelists. The rating criteria used by the panelists were as follows: zero point, no unpleasant odor; 1 point, slightly unpleasant odor; 2 point, distinct unpleasant odor; 3 point, strong unpleasant odor. Total value of the points determined by the ten panelists was used for evaluation.

The injection molding of the obtained pellet was carried out at a resin temperature of 260°C and a mold temperature of 40°C to give a test piece. Odor evaluation by dry and wet methods, measurement of aldehyde generation and taste evaluation was performed by the following methods using the obtained injection-molded product. The result is shown in Table 1. In addition, measurement of crystallization temperature (Tc) and a haze value (%) was performed for evaluating the nucleating agent performance.

### Method of measuring crystallization temperature (Tc)

According to JIS K7121, the crystallization temperature was measured using a differential scanning calorimeter (trade name "DSC7" from Perkin Elmer). The higher the To value, the quicker the crystallization rate is, and the molding cycle can be shortened.

### Haze value (improvement in clarity)

The haze value was measured using a haze meter from Toyo Seki Seisakusho according to JIS K 6714 and JIS K 6717. The smaller the measured value, the better the clarity is.

Subsequently, the obtained injection-molded product was subjected to the odor evaluation by the following dry and wet methods. The results are shown in Table 1.

### Odor evaluation of injeotion-molded product by dry method

A 225 ml glass bottle in which 20 g of test piece were sealed was left to stand in a constant temperature bath of 80°C for 2 hours, and cooled to room temperature. Immediately after that, the odor intensity was rated by ten panelists. The rating criteria used by the panelists were as follows: zero point, no unpleasant odor; 1 point, slightly unpleasant odor; 2 points, distinct unpleasant odor; 3 points, strong unpleasant odor. Total value of the points determined by the ten panelists was used for evaluation.

After sealing 20 g of test piece and 140 g of deionized water in a 225 ml glass bottle, the bottle was left in a constant temperature bath of 80°C for 2 hours and cooled to room temperature. Odor evaluations, measurement of aldehyde generation and taste evaluation was performed by the following methods using the obtained solution as an evaluation sample.

### Odor evaluation of injection-molded product by wet method

The evaluation sample was subjected to the odor intensity rating by ten panelists. The rating criteria used by the panelists were as follows: zero point, no unpleasant odor; 1 point, slightly unpleasant odor; 2 points, distinct unpleasant odor; 3 points, strong unpleasant odor. Total value of the points determined by the ten panelists was used for evaluation.

### Aldehyde generation in injection-molded product

Aldehyde content of the evaluation sample was measured using a high performance liquid chromatography. The aldehyde amount was expressed in µg/PPg, i.e. µg per 1g of test piece.

### Taste evaluation of injection-molded product

Ten gram of the evaluation sample was placed in a cup made of glass and subjected to the taste evaluation by ten panelists. In the taste evaluation, ten panelists evaluated the evaluation sample in comparison with control samples prepared by the following method, wherein the rating criteria used by the panelists were as follows: zero point, no difference; 1 point, slight difference; 2 points, distinct difference. Total value of the points determined by the ten panelists was used for evaluation.

### <Preparation of control samples for taste evaluation>

The solutions as control samples for taste evaluation were prepared in the same manner as in Comparative Example, 1 except that DBSs were not added.

In the diacetal compositions shown in Table 1, the amount of component (B) is expressed as a percentage (% by weight) based on the total amount of components (A) and (B). Accordingly, the percentage of component (A), MA (wt%), can be calculated as follows: MA = 100-MB, wherein MB is the percentage of component (B) (wt%).

**Table 1**

| Comp. Ex. | Resin | Diacetal composition | | | | | Crystallization temp. (°C) | Haze value (%) | Odor evaluation | | | Aldehyde generation (µg/PPg) | Taste evaluation |
|---|---|---|---|---|---|---|---|---|---|---|---|---|---|
| | | Component (A) | Component (B) | Amount (wt.%) | Component (C) | Amount (wt.%) | | | Pellet | Injection-molded product | | | |
| | | | | | | | | | Dry method | Dry method | Wet method | | |
| 1 | r-PP | Me-DBS | lauryl alcohol | 2.5 | - | - | 127 | 12 | 8 | 10 | 9 | 4.0 | 10 |
| 2 | r-PP | Me-DBS | myristyl alcohol | 2.5 | - | - | 128 | 13 | 8 | 9 | 9 | 4.2 | 10 |
| 3 | r-PP | Me-DBS | palmityl alcohol | 2.5 | - | - | 128 | 12 | 8 | 9 | 9 | 4.1 | 10 |
| 4 | r-PP | Me-DBS | stearyl alcohol | 2.5 | - | - | 127 | 13 | 8 | 10 | 9 | 4.0 | 10 |
| 5 | r-PP | Me-DBS | 12-hydroxystearic acid | 2.5 | - | - | 128 | 11 | 7 | 9 | 8 | 3.7 | 9 |

### EXAMPLES 1-22

Evaluation was conducted in the same manner as in Comparative Examples 1-5, except that fatty acid alkali metal salts, sulfuric ester salts and amines were employed in the amounts shown in Tables 2 and 3 in addition to the alcohols or 12-hydroxystearic acid shown in Tables 2 and 3. The results are shown in Tables 2 and 3.

In the diacetal compositions shown in Tables 2 and 3, the amount of component (B) and component (C) are expressed as a percentage (% by weight) based on the total amount of components (A),(B) and (C). Accordingly, the percentage of component (A), MA (wt%), can be calculated as follows: MA = 100-(MB+MC), wherein MB is the percentage of component (B) (wt%) and MC is the percentage of component (C) (wt%), with the proviso that MC=0, if component (C) was not used. The same applies to the following Tables 4 and 5.

**Table 2**

| Ex. | Resin | Diacetal composition | | | | | Crystallization temp. (°C) | Haze value (%) | Odor evaluation | | | Aldehyde generation (µg/PPg) | Taste evaluation |
|---|---|---|---|---|---|---|---|---|---|---|---|---|---|
| | | Component (A) | Component (B) | Amount (wt.%) | Component (C) | Amount (wt.%) | | | Pellet | Injection-molded product | | | |
| | | | | | | | | | Dry method | Dry method | Wet method | | |
| 1 | r-PP | Me-DBS | lauryl alcohol | 2.5 | Na stearate | 2.5 | 127 | 12 | 5 | 5 | 5 | 3.1 | 5 |
| 2 | r-PP | Me-DBS | myristyl alcohol | 2.5 | Na stearate | 2.5 | 128 | 12 | 4 | 5 | 4 | 3.0 | 5 |
| 3 | r-PP | Me-DBS | palmityl alcohol | 2.5 | Na stearate | 2.5 | 127 | 13 | 6 | 5 | 5 | 2.9 | 5 |
| 4 | r-PP | Me-DBS | stearyl alcohol | 2.5 | Na stearate | 2.5 | 127 | 12 | 5 | 4 | 5 | 3,0 | 5 |
| 5 | r-PP | Me-DBS | 12-hydroxy-stearic acid | 2.5 | Na laurate | 2.5 | 128 | 12 | 4 | 5 | 5 | 2.9 | 5 |
| 6 | -PP | Me-DBS | 12-hydroxy-stearic acid | 2.5 | Na palmitate | 2.5 | 127 | 12 | 4 | 4 | 5 | 3.0 | 5 |
| 7 | r-PP | Me-DBS | 12-hydroxy-stearic acid | 2.5 | Li stearate | 2.5 | 128 | 13 | 5 | 4 | 4 | 3.0 | 5 |
| 8 | -PP | Me-DBS | 12-hydroxy-stearic acid | 2.5 | Na stearate | 2.5 | 128 | 12 | 4 | 4 | 4 | 3.0 | 5 |
| 9 | r -PP | Me-DBS | 12-hydroxy-stearic acid | 2.5 | K stearate | 2.5 | 128 | 13 | 5 | 4 | 4 | 2.9 | 5 |
| 10 | r-PP | Me-DBS | 12-hydroxystearic acid 2.5 stearic acid | 2.5 | Na 12-hydroxystearate | 2.5 | 127 | 12 | 3 | 4 | 3 | 2.6 | 4 |
| 11 | r-PP | Me-DBS | 12-hydroxy-stearic acid | 2.5 | Na behenate | 2.5 | 128 | 13 | 4 | 4 | 4 | 2.9 | 4 |

**Table 3**

| Ex. | Resin | Diacetal composition | | | | | Crystallizaton temp. (°C) | Haze value (%) | Odor evaluation | | | Aldehyde generation (µ/PPg) | Taste evaluation |
|---|---|---|---|---|---|---|---|---|---|---|---|---|---|
| | | Com ponent (A) | Component (B) | Amount (wt.%) | Component (C) | Amount (wt.%) | | | Pellet | Injection-molded product | | | |
| | | | | | | | | | Dry method | Dry method | Wet method | | |
| 12 | r-PP | Me-DBS | 12-hydroxystearic acid | 2.5 | Na montanate | 2.5 | 128 | 12 | 4 | 4 | 3 | 2.8 | 4 |
| 13 | r-PP | Me-DBS | 12-hydroxy-stearic acid | 2.5 | Na oleate | 2.5 | 127 | 13 | 4 | 3 | 4 | 2.8 | 4 |
| 14 | r-PP | Me-DBS | 12-hydroxy stearic acid | 2.5 | 2.5 Na lauryl sulfate | 2.5 | 127 | 12 | 4 | 3 | 4 | 2.7 | 4 |
| 15 | r-PP | Me-DBS | 12-hydroxystearic acid | 2.5 | K lauryl sulfate | 2.5 | 127 | 13 | 4 | 4 | 3 | 2.8 | 4 |
| 16 | r-PP | Me-DBS | 12-hydroxy-stearic acid | 2.5 | Li lauryl sulfate | 2.5 | 128 | 13 | 3 | 4 | 3 | 2.9 | 5 |
| 17 | r-PP | Me-DBS | 12-hydroxystearic acid | 2.5 | Ammonium lauryl sulfate | 2.5 | 127 | 12 | 5 | 4 | 4 | 2.7 | 4 |
| 18 | r-PP | Me-DBS | 12-hydroxystearic acid | 2.5 | K oleyl sulfate K oleyl sulfate | 2.5 | 128 | 12 | 4 | 4 | 4 | 2.8 | 4 |
| 19 | r-PP | Me-DBS | 12-hydroxystearic acid | 2.5 | Na polyoxyethylene (3 moles added) lauryl ether sulfate | 2.5 | 127 | 13 | 3 | 3 | 4 | 3.0 | 5 |
| 20 | r-PP | Me-DBS | 12-hydroxystearic acid | 2.5 | Na glyceryl monolaurate sulfate | 2.5 | 127 | 12 | 4 | 4 | 3 | 3.1 | 5 |
| 21 | r-PP | Me-DBS | 12-hydroxystearic acid | 2.5 | Diethanolamine | 2.5 | 128 | 13 | 5 | 4 | 4 | 3.3 | 5 |
| 22 | r-PP | Me-DBS | 12-hydroxystearic acid | 2.5 | Triisopropanolamine | 2.5 | 127 | 13 | 5 | 5 | 4 | 3.2 | 5 |

### COMPARATIVE EXAMPLE 6

Evaluation was conducted in the same manner as in Comparative in Example 1, except that Me-DBS not containing lauryl alcohol was used. The results are shown in Table 6.

### COMPARATIVE EXAMPLE 7 AND EXAMPLES 23 AND 24

Evaluation was conducted in the same manner as in Comparative Example 5, and Examples 8 and 14, except that 1,3:2,4-dibenzylidene sorbitol (hereinafter referred to as "DBS") was used instead of Me-DBS. The results are shown in Table 4.

### COMPARATIVE EXAMPLE 8

Evaluation was conducted in the same manner as in Comparative Example 7, except that DBS not containing 12-hydroxystearic acid was used. The results are shown in Table 6.

### COMPARATIVE EXAMPLE 9 AND EXAMPLES 25 AND 26

Evaluation was conducted in the same manner as in Comparative Example 5, and Examples 8 and 14, except that 1,3:2,4-di(p-ethylbenzylidene)sorbitol (hereinafter referred to as "Et-DBS") was used instead of Me-DBS. The results are shown in Table 4.

### COMPARATIVE EXAMPLE 10

Evaluation was conducted in the same manner as in Comparative Example 9, except that Et-DBS not containing 12-hydroxysteario acid was used. The results are shown in Table 6.

### COMPARATIVE EXAMPLE 11 AND EXAMPLES 27 AND 28

Evaluation was conducted in the same manner as in Comparative Example 5, and Examples 8 and 14, except that 1,3:2,4-di(3,4-dimethylbenzylidene)sorbitol (hereinafter referred to as "3,4-DMDBS") was used instead of Me-DBS. The results are shown in Table 4.

**Table 4**

| Comp.Ex.1 Ex. | Resin | Diacetal composition | | | | | Crystallization temp. (°C) | Haze value (%) | Odor evaluation | | | Aldehyde generation (µg/PPg) | Taste evaluation |
|---|---|---|---|---|---|---|---|---|---|---|---|---|---|
| | | Component (A) | Component (B) | Amount (wt.%) | Component (C) | Amount (wt.%) | | | Pellet | Injection-molded product | | | |
| | | | | | | | | | Dry method | Dry method | Wet method | | |
| Comp. Ex.6 | r-PP | DBS | 12-hydroxystearic acid | 2.5 | - | - | 118 | 22 | 7 | 8 | 9 | 4.1 | 7 |
| Ex.23 | r-PP | DBS | 12-hydroxystearic acid | 2.5 | Na stearate | 2.5 | 117 | 21 | 6 | 5 | 5 | 3.2 | 4 |
| Ex.24 | r-PP | DBS | 12-hydroxystearic acid | 2.5 | Na lauryl sulfate | 2.5 | 118 | 22 | 5 | 4 | 5 | 3.1 | 4 |
| Comp Ex.9 | r-PP | Et-DBS | 12-hydroxystearic acid | 2.5 | - | - | 126 | 19 | 6 | 8 | 7 | 3.9 | 6 |
| Ex.25 | r-PP | Et-DBS | 12-hydroxystearic acid | 2.5 | Na stearate | 2.5 | 125 | 19 | 5 | 5 | 4 | 3.0 | 4 |
| Ex.26 | r-PP | Et-DBS | 12-hydroxystearic acid | 2.5 | Na lauryl sulfate | 2.5 | 125 | 18 | 5 | 4 | 4 | 2.9 | 4 |
| Comp. Ex.26 | r-PP | 3,4-DMDBS | 12-hydroxystearic acid | 2.5 | - | - | 127 | 13 | 6 | 7 | 7 | 3.5 | 5 |
| Ex.27 | r-PP | 3,4-DMDBS | 12-hydroxystearic acid | 2.5 | Na stearate | 2.5 | 127 | 13 | 4 | 4 | 5 | 2.4 | 2 |
| Ex. 28 | r-PP | 3,4-DMDBS | 12-hydroxystearic acid | 2.5 | Na lauryl sulfate | 2.5 | 128 | 12 | 4 | | 4 | 2.4 | 2 |

### COMPARATIVE EXAMPLE 12

Evaluation was conducted in the same manner as in Comparative Example 11, except that 3,4-DMDBS not containing 12-hydroxystearic acid was used. The results are shown in Table 6.

### COMPARATIVE EXAMPLE 13

Evaluation was conducted in the same manner as in Comparative Example 1, except that no nucleating agent was added. The results are shown in Table 6.

### COMPARATIVE EXAMPLE 14 and EXAMPLE 29

Evaluation was conducted in the same manner as in Comparative Example 5 and Example 14 except that an isotactic homopolypropylene resin (MFR = 30 g/10 minutes, hereinafter referred to as "h-PP") was used as a resin. The results are shown in Table 5.

### COMPARATIVE EXAMPLE 15

Evaluation was conducted in the same manner as in Comparative Example 14, except that Me-DBS not containing 12-hydroxysteario acid was used. The results are shown in Table 6.

### COMPARATIVE EXAMPLE 16

Evaluation was conducted in the same manner as in Comparative Example 14, except that no nucleating agent was added. The results are shown in Table 6.

### COMPARATIVE EXAMPLE 17 and EXAMPLE 30

The dibenzylidene sorbitol nucleating agent of the present invention was prepared in the same manner as in Comparative Example 1, except that 12-hydroxystearic acid, sodium lauryl sulfate and Me-DBS were used in the amounts shown in Table 5.

To 100 weight parts of a linear low density polyethylene resin (density = 0.926 g/cm3, MFR = 20 g/10 minutes, hereinafter referred to as "LLDPE"), 0.2 weight part of the dibenzylidene sorbitol nucleating agent was added, and these components were blended in a Henschel mixer. Then the mixture was melt kneaded using a single screw extruder having a diameter of 25 mm at a temperature of 200°C and pelletized. The odor evaluation of the obtained pellets was carried out by the following dry method. The results are shown in Table 5.

### Odor evaluation of pellets by dry method

A 225 ml glass bottle in which 60 g of the pellets were sealed was left to stand in a constant temperature bath of 40°C for 2 hours, and cooled to room temperature. Immediately after that, the odor intensity was rated by ten panelists. The rating criteria used by the panelists were as follows: zero point, no unpleasant odor; 1 point, slightly unpleasant odor; 2 point, distinct unpleasant odor; 3 point, strong unpleasant odor. Total value of the points determined by the ten panelists was used for evaluation.

The injection molding of the obtained pellets was carried out at a resin temperature of 220°C and a mold temperature of 30°C to give a test piece. Crystallization temperature (Tc) and haze value (%) of the resultant injection-molded product were measured by the methods employed in Comparative Example 1. The results are shown in Table 5.

Subsequently, the obtained injection-molded product was subjected to the odor evaluation by the following dry method. The results are shown in Table 5.

### Odor evaluation of injection-molded product by dry method

A 225 ml glass bottle in which 20 g of test piece were sealed was left to stand in a constant temperature bath of 40°C for 2 hours, and cooled to room temperature. Immediately after that, the odor intensity was rated by ten panelists. The rating criteria used by the panelists were as follows: zero point, no unpleasant odor; 1 point, slightly unpleasant odor; 2 points, distinct unpleasant odor; 3 points, strong unpleasant odor. Total value of the points determined by the ten panelists was used for evaluation.

### COMPARATIVE EXAMPLE 18

Evaluation was conducted in the same manner as in Comparative Example 17, except that Me-DBS not containing 12-hydroxysteario acid was used. The results are shown in Table 6.

### COMPARATIVE EXAMPLE 19

Evaluation was conducted in the same manner as in Comparative Example 17, except that no nucleating agent was added. The results are shown in Table 6.

### COMPARATIVE EXAMPLE 20 and EXAMPLE 31

Evaluation was conducted in the same manner as in Comparative Examples 17 and Example 30, except that a high density polyethylene resin (density = 0.967 g/cm3, MFR = 6.7 g/10 minutes, hereinafter referred to as "HDPE") was used as a resin. The results are shown in Table 5.

### COMPARATIVE EXAMPLE 21

Evaluation was conducted in the same manner as in Comparative Example 20, except that Me-DBS not containing 12-hydroxystearic acid was used. The results are shown in Table 6.

### COMPARATIVE EXAMPLE 22

Evaluation was conducted in the same manner as in Comparative Example 20, except that no nucleating agent was added. The results are shown in Table 6.

**Table 5**

| Comp. Ex./Ex. | Resin | Diacetal composition | | | | | Crystallization (°C) | Haze value (%) | Odor evaluation | | | Aldehyde generation (µg/PPg) | Taste evaluation |
|---|---|---|---|---|---|---|---|---|---|---|---|---|---|
| | | Component (A) | Component (B) | Amount (wt.%) | Component (C) | Amount (wt.%) | | | Pellet | Injection-molded product | | | |
| | | | | | | | | | Dry method | Dry method | Wet method | | |
| Comp. Ex. 14 | h-PP | Me-DBS | 12-hydroxystearic acid | 2.5 | - | - | 133 | 21 | 7 | 9 | 9 | 4.2 | 8 |
| Ex.28 | h-PP | Me-DBS | 12-hydroxystearic acid | 2.5 | Na lauryl sulfate | 2.5 | 133 | 21 | 5 | 5 | 5 | 3.3 | 5 |
| Comp. Ex.17 | LLDPE | Me-DBS | 12-hydroxystearic acid | 2.5 | - | - | 112 | 30 | 7 | 8 | - | - | - |
| Ex.30 | LLDPE | Me-DBS | 12-hydroxystearic acid | 2.5 | Na lauryl sulfate | 2.5 | 111 | 29 | 6 | 5 | - | - | - |
| Comp.Ex.20 | HDPE | Me-DBS | 12-hydroxystearic acid | 2.5 | - | - | 117 | - | 6 | 7 | - | - | - |
| Ex.31 | HDPE DBS | Me- | 12-hydroxystearic | 2.5 | Na lauryl sulfate | 2.5 | 116 | - | 5 | 5 | - | - | - |

**Table 6**

| Comp. Ex. | Resin | Diacetal composition | | | | | Crystallization temp. (°C) | Haze value (%) | Odor evaluation | | | Aldehyde generation (µg/PPg) | Taste evaluation |
|---|---|---|---|---|---|---|---|---|---|---|---|---|---|
| | | Component (A) | Component (B) | Amount (wt.%) | Component (C) | Amount (Wt.%) | | | Pellet | Inection-molded product | | | |
| | | | | | | | | | Dry method | Dry method | Wet method | | |
| 6 | r-PP | Me-DBS | - | - | - | - | 128 | 11 | 19 | 20 | 19 | 6.2 | 20 |
| 8 | r-PP | DBS | - | - | - | - | 119 | 21 | 20 | 17 | 16 | 6.0 | 19 |
| 10 | r-PP | Et-DBS | - | - | - | - | 127 | 18 | 18 | 18 | 17 | 5.9 | 19 |
| 12 | r-PP | 3,4-DMDBS | - | - | - | - | 128 | 12 | 16 | 16 | 15 | 5.5 | 17 |
| 13 | r-PP | - | - | - | - | - | 104 | 71 | 5 | 5 | 4 | - | - |
| 15 | h-PP | Me-DBS | - | - | - | - | 133 | 20 | 19 | 19 | 18 | 6.3 | 20 |
| 16 | h-PP | - | - | - | - | - | 113 | 74 | 4 | 4 | 3 | - | - |
| 18 | LLDPE | Me-DBS | - | - | - | - | 112 | 29 | 18 | 17 | - | - | - |
| 19 | LLDPE | - | - | - | - | - | 104 | 53 | 4 | 4 | - | - | - |
| 21 | 21 | Me-DBS | - | - | - | - | 118 | - | 16 | 17 | - | - | - |
| 22 | HDPE | - | - | - | - | - | 112 | - | 3 | 3 | - | - | - |

## Claims

1. An agent for suppressing transfer of odor and taste originating from (A) at least one diacetal represented by the formula (1): 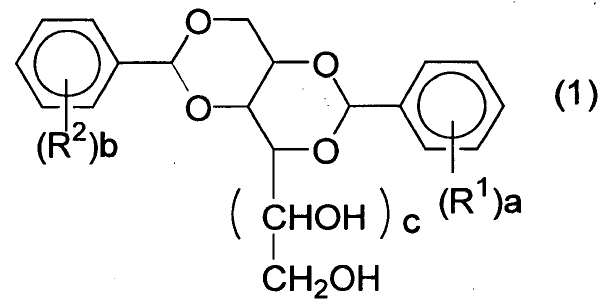 wherein R¹ and R² are the same or different and each represents a hydrogen atom, a C₁ to C₄ alkyl group, a C₁ to C₄ alkoxy group, a C₁ to C₄ alkoxycarbonyl group or a halogen atom; a and b each represents an integer of 1 to 5; c is 0 or 1; when a is 2, the two R¹ groups taken together with the benzene ring to which they are linked may form a tetralin ring; and when b is 2, the two R² groups taken together with the benzene ring to which they are linked may form a tetralin ring;
the agent comprising components (B) and (C),
wherein component (B) is at least one member selected from the group consisting of:
(B1) C₆ to C₃₂ saturated or unsaturated aliphatic alcohols; and
(B2) C₈ to C₃₂ saturated or unsaturated aliphatic carboxylic acids having at least one hydroxyl group per molecule, and
component (C) is
(C1) at least one anionic surfactant selected from the group consisting of C₆ to C₃₀ saturated or unsaturated aliphatic alcohol sulfuric ester salts, polyoxyethylene alkyl (C₈ to C₂₂) or alkenyl (C₈ to C₂₂) ether sulfuric ester salts in which the number of moles of ethylene oxide added is 1 to 8, polyoxyethylene alkyl (C₈ to C₂₂) phenyl ether sulfuric ester salts in which the number of moles of ethylene oxide added is 1 to 10, sulfuric ester salts of polyhydric alcohol fatty acid partial esters formed from a C₃ to C₆ polyhydric alcohol and a C₈ to C₂₂ saturated or unsaturated fatty acid, and C₈ to C₂₂ saturated or unsaturated fatty acid monoalkanol (C₂ to C₆) amide sulfuric ester salts, wherein the sulfuric ester salts are lithium salts, sodium salts, potassium salts and ammonium salts;
(C2) at least one member selected from the group consisting of alkali metal salts of C₈ to C₃₂ saturated or unsaturated fatty acids which may have at least one hydroxyl group per molecule;
(C3) at least one aliphatic amine selected from the group consisting of dialkanolamine, trialkanolamine, and di (C₈ to C₂₂ alkyl or alkenyl) methylamine; or
(C4) a mixture of at least two of (C1), (C2) and (C3).

2. The agent for suppressing transfer of odor and taste according to claim 1, wherein
component (B) is at least one member selected from the group consisting of 9-hydroxystearic acid, 10-hydroxystearic acid, 12-hydroxystearic acid, 9,10-dihydroxystearic acid, lauryl alcohol, myristyl alcohol, palmityl alcohol, stearyl alcohol and behenyl alcohol, and
component (C) is (C2a) at least one member selected from the group consisting of lithium salts, sodium salts and potassium salts of C₈ to C₃₂ saturated or unsaturated fatty acids which may have at least one hydroxyl group per molecule, or
component (C) is (C1a) at least one sulfuric ester salt selected from the group consisting of lauryl sulfate salts, stearyl sulfate salts, oleyl sulfate salts, polyoxyethylene (the number of moles of ethylene oxide added = 2 to 3) lauryl ether sulfate salts, polyoxyethylene (the number of moles of ethylene oxide added = 2 to 3) stearyl ether sulfate salts, polyoxyethylene (the number of moles of ethylene oxide added = 2 to 3) nonylphenyl ether sulfate salts, polyoxyethylene (the number of moles of ethylene oxide added = 2 to 3) dodecylphenyl ether sulfate salts, glyceryl monolaurate sulfate salts, glyceryl monostearate sulfate salts, lauric acid monoethanolamide sulfuric ester salts, stearic acid monoethanolamide sulfuric ester salts, and oleic acid monoethanolamide sulfuric ester salts, wherein the sulfuric ester salts or sulfate salts are lithium salts, sodium salts and potassium salts.

3. The agent for suppressing transfer of odor and taste according to claim 2, wherein component (C) is at least one member selected from the group consisting of sodium lauryl sulfate, potassium lauryl sulfate, sodium stearate, potassium stearate, sodium 12-hydroxystearate and potassium 12-hydroxystearate.

4. The agent for suppressing transfer of odor and taste according to any one of claims 1-3, wherein the weight ratio of component (B) to component (C) is 1:0.2 to 5.

5. A method for suppressing aldehyde generation by thermal decomposition of (A) at least one diacetal represented by the formula (1) : 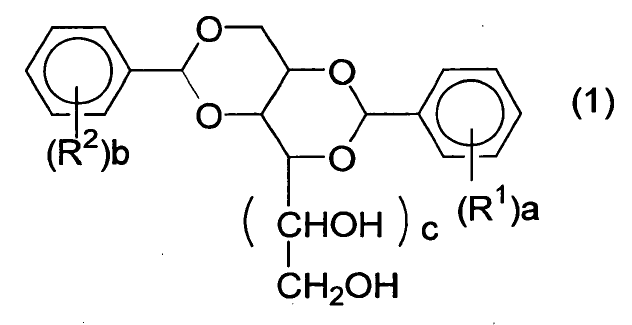 wherein R¹ and R² are the same or different and each represents a hydrogen atom, a C₁ to C₄ alkyl group, a C₁ to C₄ alkoxy group, a C₁ to C₄ alkoxycarbonyl group or a halogen atom; a and b each represents an integer of 1 to 5; c is 0 or 1; when a is 2, the two R¹ groups taken together with the benzene ring to which they are linked may form a tetralin ring; and when b is 2, the two R² groups taken together with the benzene ring to which they are linked may form a tetralin ring;
the method comprising adding the following components (B) and (C) to the diacetal,
wherein component (B) is at least one member selected from the group consisting of:
(B1) C₆ to C₃₂ saturated or unsaturated aliphatic alcohols; and
(B2) C₈ to C₃₂ saturated or unsaturated aliphatic carboxylic acids having at least one hydroxyl group per molecule, and
component (C) is
(C1) at least one anionic surfactant selected from the group consisting of C₆ to C₃₀ saturated or unsaturated aliphatic alcohol sulfuric ester salts, polyoxyethylene alkyl (C₈ to C₂₂) or alkenyl (C₈ to C₂₂) ether sulfuric ester salts in which the number of moles of ethylene oxide added is 1 to 8, polyoxyethylene alkyl (C₈ to C₂₂) phenyl ether sulfuric ester salts in which the number of moles of ethylene oxide added is 1 to 10, sulfuric ester salts of polyhydric alcohol fatty acid partial esters formed from a C₃ to C₆ polyhydric alcohol and a C₈ to C₂₂ saturated or unsaturated fatty acid, and C₈ to C₂₂ saturated or unsaturated fatty acid monoalkanol (C₂ to C₆) amide sulfuric ester salts, wherein the sulfuric ester salts are lithium salts, sodium salts, potassium salts and ammonium salts;
(C2) at least one member selected from the group consisting of alkali metal salts of C₈ to C₃₂ saturated or unsaturated fatty acids which may have at least one hydroxyl group per molecule;
(C3) at least one aliphatic amine selected from the group consisting of dialkanolamine, trialkanolamine, and di (C₈ to C₂₂ alkyl or alkenyl) methylamine; or
(C4) a mixture of at least two of (C1), (C2) and (C3).

6. The method according to claim 5, wherein component (B) is at least one member selected from the group consisting of 9-hydroxystearic acid, 10-hydroxystearic acid, 12-hydroxystearic acid, 9,10-dihydroxystearic acid, lauryl alcohol, myristyl alcohol, palmityl alcohol, stearyl alcohol and behenyl alcohol, and
component (C) is (C2a) at least one member selected from the group consisting of lithium salts, sodium salts and potassium salts of C₈ to C₃₂ saturated or unsaturated fatty acids which may have at least one hydroxyl group per molecule, or
component (C) is (C1a) at least one sulfuric ester salt selected from the group consisting of lauryl sulfate salts, stearyl sulfate salts, oleyl sulfate salts, polyoxyethylene (the number of moles of ethylene oxide added = 2 to 3) lauryl ether sulfate salts, polyoxyethylene (the number of moles of ethylene oxide added = 2 to 3) stearyl ether sulfate salts, polyoxyethylene (the number of moles of ethylene oxide added = 2 to 3) nonylphenyl ether sulfate salts, polyoxyethylene (the number of moles of ethylene oxide added = 2 to 3) dodecylphenyl ether sulfate salts, glyceryl monolaurate sulfate salts, glyceryl monostearate sulfate salts, lauric acid monoethanolamide sulfuric ester salts, stearic acid monoethanolamide sulfuric ester salts, and oleic acid monoethanolamide sulfuric ester salts, wherein the sulfuric ester salts or sulfate salts are lithium salts, sodium salts and potassium salts.

7. The method according to claim 6, wherein component (C) is at least one member selected from the group consisting of sodium lauryl sulfate, potassium lauryl sulfate, sodium stearate, potassium stearate, sodium 12-hydroxystearate and potassium 12-hydroxystearate.

8. The method according to any one of claims 5-7, wherein the weight ratio of component (B) to component (C) is 1:0.2 to 5.

9. A granular or powdery diacetal composition wherein transfer of odor and taste originating from the diacetal is suppressed;
the composition comprising components (A), (B) and (C), wherein component (A) is at least one diacetal represented by the formula (1) 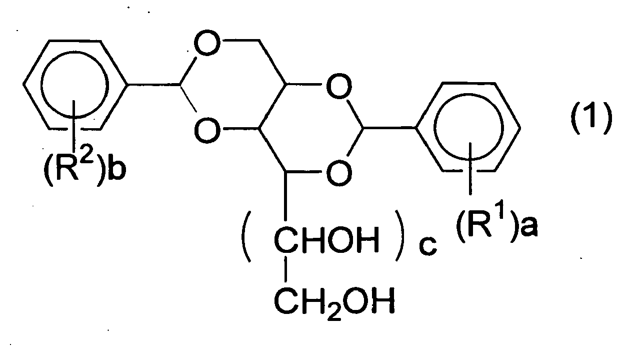 wherein R¹ and R² are the same or different and each represents a hydrogen atom, a C₁ to C₄ alkyl group, a C₁ to C₄ alkoxy group, a C₁ to C₄ alkoxycarbonyl group or a halogen atom; a and b each represents an integer of 1 to 5; c is 0 or 1; when a is 2, the two R¹ groups taken together with the benzene ring to which they are linked may form a tetralin ring; and when b is 2, the two R² groups taken together with the benzene ring to which they are linked may form a tetralin ring,
component (B) is at least one member selected from the group consisting of:
(B1) C₆ to C₃₂ saturated or unsaturated aliphatic alcohols; and
(B2) C₈ to C₃₂ saturated or unsaturated aliphatic carboxylic acids having at least one hydroxyl group per molecule, and
component (C) is
(C1) at least one anionic surfactant selected from the group consisting of C₆ to C₃₀ saturated or unsaturated aliphatic alcohol sulfuric ester salts, polyoxyethylene alkyl (C₈ to C₂₂) or alkenyl (C₈ to C₂₂) ether sulfuric ester salts in which the number of moles of ethylene oxide added is 1 to 8, polyoxyethylene alkyl (C₈ to C₂₂) phenyl ether sulfuric ester salts in which the number of moles of ethylene oxide added is 1 to 10, sulfuric ester salts of polyhydric alcohol fatty acid partial esters formed from a C₃ to C₆ polyhydric alcohol and a C₈ to C₂₂ saturated or unsaturated fatty acid, and C₈ to C₂₂ saturated or unsaturated fatty acid monoalkanol (C₂ to C₆) amide sulfuric ester salts, wherein the sulfuric ester salts are lithium salts, sodium salts, potassium salts and ammonium salts;
(C2) at least one member selected from the group consisting of alkali metal salts of C₈ to C₃₂ saturated or unsaturated fatty acids which may have at least one hydroxyl group per molecule;
(C3) at least one aliphatic amine selected from the group consisting of dialkanolamine, trialkanolamine, and di(C₈ to C₂₂ alkyl or alkenyl) methylamine; or
(C4) a mixture of at least two of (C1), (C2) and (C3).

10. The diacetal composition according to claim 9, wherein based on the total amount of components (A), (B) and (C), component (B) is present in a proportion of 0.1 to 5 wt% and component (C) is present in a proportion of 0.1 to 5 wt%.

11. The diacetal composition according to claim 10, wherein the weight ratio of component (B) to component (C) is 1:0.2 to 5.

12. A polyolefin resin nucleating agent comprising the diacetal composition according to any one of claims 9 to 11, wherein transfer of odor and taste originating from the diacetal is suppressed.

13. A polyolefin resin composition comprising the polyolefin resin nucleating agent according to claim 12 and a polyolefin resin, wherein transfer of odor and taste originating from the diacetal is suppressed.

14. The polyolefin resin composition according to claim 13, wherein the polyolefin resin nucleating agent according to claim 12 is present in an amount of 0.05 to 3 weight parts per 100 weight parts of the polyolefin resin.

15. A polyolefin resin molded product prepared by molding the polyolefin resin composition according to claim 13 or 14, wherein transfer of odor and taste originating from the diacetal is suppressed.

16. A container or a packaging material for foods, cosmetics or medicines comprising the polyolefin resin molded product according to claim 15, wherein transfer of odor and taste originating from the diacetal is suppressed.

17. A method for suppressing odor originating from a diacetal at the time of molding a polyolefin resin, comprising mixing the nucleating agent according to claim 12 with a polyolefin resin and molding a resultant resin composition.

18. A method for suppressing transfer of odor and taste originating from a diacetal to a content (such as foods, cosmetics and medicines), **characterized in that** it comprises placing the content in a packaging material or a container prepared by mixing the nucleating agent according to claim 12 with a polyolefin resin and molding a resultant resin composition.

## Patentansprüche

1. Mittel zum Unterdrücken des Transfers von Geruch und Geschmack, die von (A) mindestens einem durch die Formel (1) dargestellten Diacetal stammen: 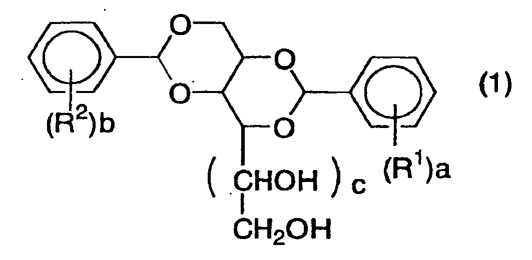 worin R¹ und R² gleich oder verschieden sind und jeweils ein Wasserstoffatom, eine C₁-C₄-Alkylgruppe, eine C₁-C₄-Alkoxygruppe, eine C₁-C₄-Alkoxycarbonylgruppe oder ein Halogenatom darstellen; a und b jeweils eine ganze Zahl von 1 bis 5 sind; c 0 oder 1 ist; wenn a 2 ist, die beiden Gruppen R¹ zusammengenommen mit dem Benzolring, an dem sie verknüpft sind, einen Tetralinring bilden können; und, wenn b 2 ist, die beiden Gruppen R² zusammengenommen mit dem Benzolring, an dem sie verknüpft sind, einen Tetralinring bilden können;
wobei das Mittel die Komponenten (B) und (C) umfasst,
wobei Komponente (B) mindestens ein Element ausgewählt aus der Gruppe bestehend aus:
(B1) gesättigten oder ungesättigten aliphatischen C₆-C₃₂-Alkoholen; und
(B2) gesättigten oder ungesättigten aliphatischen C₈-C₃₂-Carbonsäuren mit mindestens einer Hydroxylgruppe pro Molekül ist, und Komponente (C)
(C1) mindestens ein anionisches Tensid ausgewählt aus der Gruppe bestehend aus gesättigten oder ungesättigten aliphatischen C₆-C₃₀-Alkohol-schwefelsäureestersalzen, Polyoxyethylenalkyl (C₈ bis C₂₂)- oder -alkenyl (C₈ bis C₂₂)-etherschwefelsäureestersalzen, in denen die Molanzahl an addiertem Ethylenoxid 1 bis 8 ist, Polyoxyethylenalkyl (C₈ bis C₂₂)-phenyletherschwefelsäureestersalzen, bei denen die Molanzahl an addiertem Ethylenoxid 1 bis 10 ist, Schwefelsäureestersalzen von mehrwertigen Alkoholfettsäurepartialestern gebildet aus einem mehrwertigen C₃-C₆-Alkohol und einer gesättigten oder ungesättigten C₈-C₂₂-Fettsäure und gesättigten oder ungesättigten C₈-C₂₂-Fettsäuremonoalkanol (C₂ bis C₆)-amidschwefelsäureestersalzen, wobei die Schwefelsäureestersalze Lithiumsalze, Natriumsalze, Kaliumsalze und Ammoniumsalze sind;
(C2) mindestens ein Element ausgewählt aus der Gruppe bestehend aus Alkalimetallsalzen von gesättigten oder ungesättigten C₈-C₃₂-Fettsäuren, die mindestens eine Hydroxylgruppe pro Molekül aufweisen können;
(C3) mindestens ein aliphatisches Amin ausgewählt aus der Gruppe bestehend aus Dialkanolamin, Trialkanolamin und Di(C₈-C₂₂-alkyl- oder -alkenyl)methylamin; oder
(C4) eine Mischung von mindestens zwei von (C1), (C2) und (C3) ist.

2. Mittel zum Unterdrücken des Transfers von Geruch und Geschmack nach Anspruch 1, worin
Komponente (B) mindestens ein Element ausgewählt aus der Gruppe bestehend aus 9-Hydroxystearinsäure, 10-Hydroxystearinsäure, 12-Hydroxystearinsäure, 9,10-Dihydroxystearinsäure, Laurylalkohol, Myristylalkohol, Palmitylalkohol, Stearylalkohol und Behenylalkohol ist und
Komponente (C) (C2a) mindestens ein Element ausgewählt aus der Gruppe bestehend aus Lithiumsalzen, Natriumsalzen und Kaliumsalzen von gesättigten oder ungesättigten C₈-C₃₂-Fettsäuren, die mindestens eine Hydroxylgruppe pro Molekül aufweisen können, ist oder
Komponente (C) (C1a) mindestens ein Schwefelsäureestersalz, das ausgewählt ist aus der Gruppe bestehend aus Laurylsulfatsalzen, Stearylsulfatsalzen, Oleylsulfatsalzen, Polyoxyethylenlaurylethersulfatsalzen (die Molanzahl von addiertem Ethylenoxid = 2 bis 3), Polyoxyethylenstearylethersulfatsalzen (die Molanzahl an addiertem Ethylenoxid = 2 bis 3), Polyoxyethylennonylphenylethersulfatsalzen (die Molanzahl von addiertem Ethylenoxid = 2 bis 3), Polyoxyethylendodecylphenylethersulfatsalzen (die Molanzahl an addiertem Ethylenoxid = 2 bis 3), Glycerylmonolauratsulfatsalzen, Glycerylmonostearatsulfatsalzen, Laurinsäuremonoethanolamidschwefelsäureestersalzen, Stearinsäuremonoethanolamidschwefelsäureestersalzen und Ölsäuremonoethanolamidschwefelsäureestersalzen, wobei die Schwefelsäureestersalze oder die Sulfatsalze Lithiumsalze, Natriumsalze und Kaliumsalze sind, ist.

3. Mittel zum Unterdrücken des Transfers von Geruch und Geschmack nach Anspruch 2, worin Komponente (C) mindestens ein Element ausgewählt aus der Gruppe bestehend aus Natriumlaurylsulfat, Kaliumlaurylsulfat, Natriumstearat, Kaliumstearat, Natrium-12-hydroxystearat und Kalium-12-hydroxy-stearat ist.

4. Mittel zum Unterdrücken des Transfers von Geruch und Geschmack nach irgendeinem der Ansprüche 1 bis 3, worin das Gewichtsverhältnis von Komponente (B) zu Komponente (C) 1 : 0,2 bis 5 ist.

5. Verfahren zum Unterdrücken der Aldehydbildung durch thermische Zersetzung von (A) mindestens einem durch die Formel (1) dargestellten Diacetal: 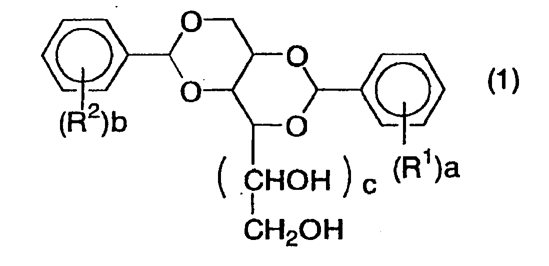 worin R¹ und R² gleich oder verschieden sind und jeweils ein Wasserstoffatom, eine C₁-C₄-Alkylgruppe, eine C₁-C₄-Alkoxygruppe, eine C₁-C₄-Alkoxycarbonylgruppe oder ein Halogenatom darstellen; a und b jeweils eine ganze Zahl von 1 bis 5 sind; c 0 oder 1 ist; wenn a 2 ist, die beiden Gruppen R¹ zusammengenommen mit dem Benzolring, an dem sie verknüpft sind, einen Tetralinring bilden können; und, wenn b 2 ist, die beiden Gruppen R² zusammengenommen mit dem Benzolring, an dem sie verknüpft sind, einen Tetralinring bilden können;
wobei das Verfahren die Zugabe der folgenden Komponenten (B) und (C) zum Diacetal umfasst,
wobei Komponente (B) mindestens ein Element ausgewählt aus der Gruppe bestehend aus:
(B1) gesättigten oder ungesättigten aliphatischen C₆-C₃₂-Alkoholen; und
(B2) gesättigten oder ungesättigten aliphatischen C₈-C₃₂-Carbonsäuren mit mindestens einer Hydroxylgruppe pro Molekül ist, und Komponente (C)
(C1) mindestens ein anionisches Tensid ausgewählt aus der Gruppe bestehend aus gesättigten oder ungesättigten aliphatischen C₆-C₃₀-Alkoholschwefelsäureestersalzen, Polyoxyethylenalkyl (C₈ bis C₂₂)- oder -alkenyl (C₈ bis C₂₂)-etherschwefelsäureestersalzen, in denen die Molanzahl an addiertem Ethylenoxid 1 bis 8 ist, Polyoxyethylenalkyl (C₈ bis C₂₂)-phenyletherschwefelsäureestersalzen, bei denen die Molanzahl an addiertem Ethylenoxid 1 bis 10 ist, Schwefelsäureestersalzen von mehrwertigen Alkoholfettsäurepartialestern gebildet aus einem mehrwertigen C₃-C₆-Alkohol und einer gesättigten oder ungesättigten C₈-C₂₂-Fettsäure und gesättigten oder ungesättigten C₈-C₂₂-Fettsäuremonoalkanol (C₂ bis C₆)-amidschwefelsäureestersalzen, wobei die Schwefelsäureestersalze Lithiumsalze, Natriumsalze, Kaliumsalze und Ammoniumsalze sind;
(C2) mindestens ein Element ausgewählt aus der Gruppe bestehend aus Alkalimetallsalzen von gesättigten oder ungesättigten C₈-C₃₂-Fettsäuren, die mindestens eine Hydroxylgruppe pro Molekül aufweisen können;
(C3) mindestens ein aliphatisches Amin ausgewählt aus der Gruppe bestehend aus Dialkanolamin, Trialkanolamin und Di(C₈-C₂₂-alkyl- oder -alkenyl)methylamin; oder
(C4) eine Mischung von mindestens zwei von (C1), (C2) und (C3) ist.

6. Verfahren nach Anspruch 5, worin Komponente (B) mindestens ein Element ausgewählt aus der Gruppe bestehend aus 9-Hydroxystearinsäure, 10-Hydroxystearinsäure, 12-Hydroxystearinsäure, 9,10-Dihydroxystearinsäure, Laurylalkohol, Myristylalkohol, Palmitylalkohol, Stearylalkohol und Behenylalkohol ist und
Komponente (C) (C2a) mindestens ein Element ausgewählt aus der Gruppe bestehend aus Lithiumsalzen, Natriumsalzen und Kaliumsalzen von gesättigten oder ungesättigten C₈-C₃₂-Fettsäuren, die mindestens eine Hydroxylgruppe pro Molekül aufweisen können, ist oder
Komponente (C) (C1a) mindestens ein Schwefelsäureestersalz, das ausgewählt ist aus der Gruppe bestehend aus Laurylsulfatsalzen, Stearylsulfatsalzen, Oleylsulfatsalzen, Polyoxyethylenlaurylethersulfatsalzen (die Molanzahl von addiertem Ethylenoxid = 2 bis 3), Polyoxyethylenstearylethersulfatsalzen (die Molanzahl an addiertem Ethylenoxid = 2 bis 3), Polyoxyethylennonylphenylethersulfatsalzen (die Molanzahl von addiertem Ethylenoxid = 2 bis 3), Polyoxyethylendodecylphenylethersulfatsalzen (die Molanzahl an addiertem Ethylenoxid = 2 bis 3), Glycerylmonolauratsulfatsalzen, Glycerylmonostearatsulfatsalzen, Laurinsäuremonoethanolamidschwefelsäureestersalzen, Stearinsäuremonoethanolamidschwefelsäureestersalzen und Ölsäuremonoethanolamidschwefelsäureestersalzen, wobei die Schwefelsäureestersalze oder die Sulfatsalze Lithiumsalze, Natriumsalze und Kaliumsalze sind, ist.

7. Verfahren nach Anspruch 6, worin Komponente (C) mindestens ein Element ausgewählt aus der Gruppe bestehend aus Natriumlaurylsulfat, Kaliumlaurylsulfat, Natriumstearat, Kaliumstearat, Natrium-12-hydroxystearat und Kalium-12-hydroxystearat ist.

8. Verfahren nach irgendeinem der Ansprüche 5 bis 7, worin das Gewichtsverhältnis von Komponente (B) zu Komponente (C) 1: 0,2 bis 5 ist.

9. Granuläre oder pulvrige Diacetalzusammensetzung, wobei der Transfer von Geruch und Geschmack, die von dem Diacetal stammen, unterdrückt ist; wobei die Zusammensetzung die Komponenten (A), (B) und (C) umfasst, wobei Komponente (A) mindestens ein durch die Formel (1) dargestelltes Diacetal ist: 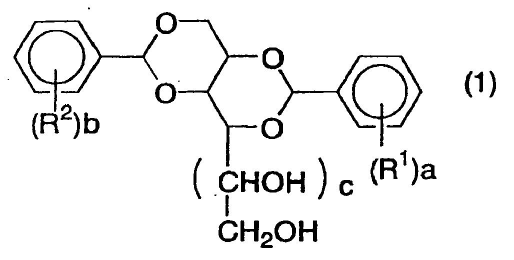 worin R¹ und R² gleich oder verschieden sind und jeweils ein Wasserstoffatom, eine C₁-C₄-Alkylgruppe, eine C₁-C₄-Alkoxygruppe, eine C₁-C₄-Alkoxycarbonylgruppe oder ein Halogenatom darstellen; a und b jeweils eine ganze Zahl von 1 bis 5 sind; c 0 oder 1 ist; wenn a 2 ist, die beiden Gruppen R¹ zusammengenommen mit dem Benzolring, an dem sie verknüpft sind, einen Tetralinring bilden können; und, wenn b 2 ist, die beiden Gruppen R² zusammengenommen mit dem Benzolring, an dem sie verknüpft sind, einen Tetralinring bilden können;
die Komponente (B) mindestens ein Element ausgewählt aus der Gruppe bestehend aus:
(B1) gesättigten oder ungesättigten aliphatischen C₆-C₃₂-Alkoholen; und
(B2) gesättigten oder ungesättigten aliphatischen C₈-C₃₂-Carbonsäuren mit mindestens einer Hydroxylgruppe pro Molekül ist, und Komponente (C)
(C1) mindestens ein anionisches Tensid ausgewählt aus der Gruppe bestehend aus gesättigten oder ungesättigten aliphatischen C₆-C₃₀-Alkoholschwefelsäureestersalzen, Polyoxyethylenalkyl (C₈ bis C₂₂)- oder -alkenyl (C₈ bis C₂₂)-etherschwefelsäureestersalzen, in denen die Molanzahl an addiertem Ethylenoxid 1 bis 8 ist, Polyoxyethylenalkyl (C₈ bis C₂₂)-phenyletherschwefelsäureestersalzen, bei denen die Molanzahl an addiertem Ethylenoxid 1 bis 10 ist, Schwefelsäureestersalzen von mehrwertigen Alkoholfettsäurepartialestern gebildet aus einem mehrwertigen C₃-C₆-Alkohol und einer gesättigten oder ungesättigten C₈-C₂₂-Fettsäure und gesättigten oder ungesättigten C₈-C₂₂-Fettsäuremonoalkanol (C₂ bis C₆)-amidschwefelsäureestersalzen, wobei die Schwefelsäureestersalze Lithiumsalze, Natriumsalze, Kaliumsalze und Ammoniumsalze sind;
(C2) mindestens ein Element ausgewählt aus der Gruppe bestehend aus Alkalimetallsalzen von gesättigten oder ungesättigten C₈-C₃₂-Fettsäuren, die mindestens eine Hydroxylgruppe pro Molekül aufweisen können;
(C3) mindestens ein aliphatisches Amin ausgewählt aus der Gruppe bestehend aus Dialkanolamin, Trialkanolamin und Di(C₈-C₂₂-alkyl- oder -alkenyl)methylamin; oder
(C4) eine Mischung von mindestens zwei von (C1), (C2) und (C3) ist.

10. Diacetalzusammensetzung nach Anspruch 9, worin, bezogen auf die Gesamtmenge der Komponenten (A), (B) und (C), Komponente (B) in einem Anteil von 0,1 bis 5 Gew.-% vorhanden ist und Komponente (C) in einem Anteil von 0,1 bis 5 Gew.-% vorhanden ist.

11. Diacetalzusammensetzung nach Anspruch 10, worin das Gewichtsverhältnis von Komponente (B) zu Komponente (C) 1: 0,2 bis 5 ist.

12. Polyolefinharz-Nukleierungsmittel, umfassend die Diacetalzusammensetzung nach irgendeinem der Ansprüche 9 bis 11, wobei der Transfer von Geruch und Geschmack, die von dem Diacetal stammen, unterdrückt ist.

13. Polyolefinharz-Zusammensetzung, umfassend das Polyolefinharz-Nukleierungsmittel nach Anspruch 12 und ein Polyolefinharz, wobei der Transfer von Geruch und Geschmack, die von dem Diacetal stammen, unterdrückt ist.

14. Polyolefinharz-Zusammensetzung nach Anspruch 13, wobei das Polyolefinharz-Nukleierungsmittel nach Anspruch 12 in einer Menge von 0,05 bis 3 Gew.-Teilen pro 100 Gew.-Teilen des Polyolefinharzes vorhanden ist.

15. Polyolefinharz-Formteil, hergestellt durch Formen der Polyolefinharz-Zusammensetzung nach Anspruch 13 oder 14, wobei der Transfer von Geruch und Geschmack, die von dem Diacetal stammen, unterdrückt ist.

16. Behälter oder Verpackungsmaterial für Nahrungsmittel, Kosmetika oder Arzneimittel, umfassend das Polyolefinharz-Formteil nach Anspruch 15, wobei der Transfer von Geruch und Geschmack, die von dem Diacetal stammen, unterdrückt ist.

17. Verfahren zum Unterdrücken von Geruch, der von einem Diacetal stammt, zum Zeitpunkt des Formens eines Polyolefinharzes, umfassend das Mischen des Nukleierungsmittels nach Anspruch 12 mit einem Polyolefinharz und das Formen einer sich ergebenden Harzzusammensetzung.

18. Verfahren zum Unterdrücken des Transfers von Geruch und Geschmack, die von einem Diacetal stammen, auf einen Inhalt (wie Nahrungsmittel, Kosmetika und Arzneimittel), **dadurch gekennzeichnet, dass** es das Unterbringen des Inhalts in ein Verpackungsmaterial oder einen Behälter umfasst, das bzw. der durch Mischen des Nukleierungsmittels nach Anspruch 12 mit einem Polyolefinharz und Formen einer sich ergebenden Harzzusammensetzung hergestellt wird.

## Revendications

1. Agent permettant de supprimer le transfert d'odeur et de goût provenant de (A) au moins un diacétal représenté par la formule (1) _{:} 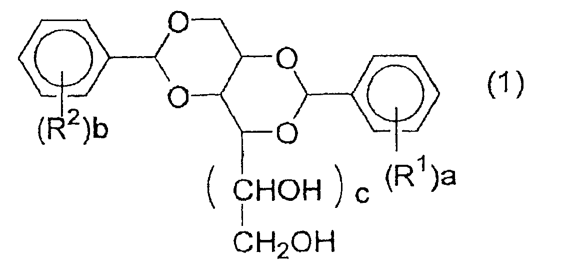 dans laquelle R¹ et R² sont identiques ou différents et chacun représente un atome d'hydrogène, un groupe alkyle en C₁ à C₄,
un groupe alcoxy en C₁ à C₄, un groupe alcoxycarbonyle en C₁ à C₄ ou un atome d'halogène ; a et b représentent chacun un nombre entier de 1 à 5 ; c est 0 ou 1 ; quand a vaut 2, les deux groupes R¹ pris ensemble avec le cycle benzénique auquel ils sont liés peuvent former un cycle tétraline ; et quand b vaut 2, les deux groupes R² pris ensemble avec le cycle benzénique auquel ils sont liés peuvent former un cycle tétraline ;
l'agent comprenant les composants (B) et (C),
où le composant (B) est au moins un élément choisi dans le groupe constitué par :
(B1) les alcools aliphatiques en C₆ à C₃₂ saturés ou non ;
et
(B2) les acides carboxyliques aliphatiques en C₈ à C₃₂ saturés ou non ayant au moins un groupe hydroxyle par molécule, et
le composant (C) est
(C1) au moins un tensioactif anionique choisi dans le groupe constitué par les sels d'esters sulfuriques des alcools aliphatiques en C₆ à C₃₀ saturés ou non, les sels d'esters sulfuriques des éthers d'alkyle (C₈ à C₂₂) ou d'alcényle (C₈ à C₂₂) de polyoxyéthylènes dans lesquels le nombre de moles d'oxyde d'éthylène ajoutées est de 1 à 8, les sels d'esters sulfuriques des éthers d'alkyl-(C₈ à C₂₂)-phényle (C₈ à C₂₂) de polyoxyéthylènes dans lesquels le nombre de moles d'oxyde d'éthylène ajoutées est de 1 à 10, les sels d'esters sulfuriques des esters partiels d'acides gras de polyols formés à partir d'un polyol en C₃ à C₆ et d'un acide gras en C₈ à C₂₂ saturé ou non, et les sels d'esters sulfuriques des amides de monoalcanol (C₂ à C₆) d'acides gras en C₈ à C₂₂ saturés ou non, dans lesquels les sels d'esters sulfuriques sont les sels de lithium, les sels de sodium, les sels de potassium et les sels d'ammonium ;
(C2) au moins un élément choisi dans le groupe constitué par les sels de métal alcalin des acides gras en C₈ à C₃₂ saturés ou non qui peuvent avoir au moins un groupe hydroxyle par molécule ;
(C3) au moins une amine aliphatique choisie dans le groupe constitué par la dialcanolamine, la trialcanolamine et la di(alkyl- ou alcényl- C₈ à C₂₂)méthylamine ; ou
(C4) un mélange d'au moins deux des (C1), (C2) et (C3).

2. Agent permettant de supprimer le transfert d'odeur et de goût selon la revendication 1, dans lequel
le composant (B) est au moins un élément choisi dans le groupe constitué par l'acide 9-hydroxystéarique, l'acide 10-hydroxystéarique, l'acide 12-hydroxystéarique, l'acide 9,10-dihydroxystéarique, l'alcool laurylique, l'alcool myristylique, l'alcool palmitylique, l'alcool stéarylique et l'alcool béhénylique, et
le composant (C) est (C2a) au moins un élément choisi dans le groupe constitué par les sels de lithium, les sels de sodium et les sels de potassium des acides gras en C₈ à C₃₂ saturés ou non qui peuvent avoir au moins un groupe hydroxyle par molécule, ou
le composant (C) est (C1a) au moins un sel d'ester sulfurique choisi dans le groupe constitué par les sels de sulfate de lauryle, les sels de sulfate de stéaryle, les sels de sulfate d'oléyle, les sels de sulfate de l'éther laurylique de polyoxyéthylène (nombre de moles d'oxyde d'éthylène ajoutées = 2 à 3), les sels de sulfate de l'éther stéarylique de polyoxyéthylène (nombre de moles d'oxyde d'éthylène ajoutées = 2 à 3), les sels de sulfate de l'éther nonylphénylique de polyoxyéthylène (nombre de moles d'oxyde d'éthylène ajoutées = 2 à 3), les sels de sulfate de l'éther dodécylphénylique de polyoxyéthylène (nombre de moles d'oxyde d'éthylène ajoutées = 2 à 3), les sels de sulfate du monolaurate de glycéryle, les sels de sulfate du monostéarate de glycéryle, les sels d'esters sulfuriques des monoéthanolamides d'acide laurique, les sels d'esters sulfuriques des monoéthanolamides d'acide stéarique et les sels d'esters sulfuriques des monoéthanolamides d'acide oléique, dans lesquels les sels d'esters sulfuriques ou les sels de sulfate sont les sels de lithium, les sels de sodium et les sels de potassium.

3. Agent permettant de supprimer le transfert d'odeur et de goût selon la revendication 2, dans lequel le composant (C) est au moins un élément choisi dans le groupe constitué par le laurylsulfate de sodium, le laurylsulfate de potassium, le stéarate de sodium, le stéarate de potassium, le 12-hydroxystéarate de sodium et le 12-hydroxystéarate de potassium.

4. Agent permettant de supprimer le transfert d'odeur et de goût selon l'une quelconque des revendications 1 à 3, dans lequel le rapport en poids du composant (B) au composant (C) est de 1/0,2 à 5.

5. Procédé permettant de supprimer la formation d'aldéhyde par décomposition thermique de (A) au moins un diacétal représenté par la formule (1) : 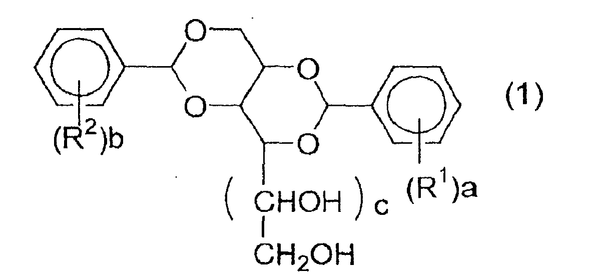 dans laquelle R¹ et R² sont identiques ou différents et chacun représente un atome d'hydrogène, un groupe alkyle en C₁ à C₄, un groupe alcoxy en C₁ à C₄, un groupe alcoxycarbonyle en C₁ à C₄ ou un atome d'halogène ; a et b représentent chacun un nombre entier de 1 à 5 ; c est 0 ou 1 ; quand a vaut 2, les deux groupes R¹ pris ensemble avec le cycle benzénique auquel ils sont liés peuvent former un cycle tétraline ; et quand b vaut 2, les deux groupes R² pris ensemble avec le cycle benzénique auquel ils sont liés peuvent former un cycle tétraline ;
le procédé comprenant l'ajout des composants (B) et (C) suivants au diacétal,
où le composant (B) est au moins un élément choisi dans le groupe constitué par :
(B1) les alcools aliphatiques en C₆ à C₃₂ saturés ou non ; et
(B2) les acides carboxyliques aliphatiques en C₈ à C₃₂ saturés ou non ayant au moins un groupe hydroxyle par molécule, et
le composant (C) est
(C1) au moins un tensioactif anionique choisi dans le groupe constitué par les sels d'esters sulfuriques des alcools aliphatiques en C₆ à C₃₀ saturés ou non, les sels d'esters sulfuriques des éthers d'alkyle (C₈ à C₂₂) ou d'alcényle (C₈ à C₂₂) de polyoxyéthylènes dans lesquels le nombre de moles d'oxyde d'éthylène ajoutées est de 1 à 8, les sels d'esters sulfuriques des éthers d'alkyl-(C₈ à C2₂)-phényle (C₈ à C₂₂) de polyoxyéthylènes dans lesquels le nombre de moles d'oxyde d'éthylène ajoutées est de 1 à 10, les sels d'esters sulfuriques des esters partiels d'acides gras de polyols formés à partir d'un polyol en C₃ à C₆ et d'un acide gras en C₈ à C₂₂ saturé ou non et les sels d'esters sulfuriques des amides de monoalcanol (C₂ à C₆) d'acides gras en C₈ à C₂₂ saturés ou non, dans lesquels les sels d'esters sulfuriques sont les sels de lithium, les sels de sodium, les sels de potassium et les sels d'ammonium ;
(C2) au moins un élément choisi dans le groupe constitué par les sels de métal alcalin des acides gras en C₈ à C₃₂ saturés ou non qui peuvent avoir au moins un groupe hydroxyle par molécule ;
(C3) au moins une amine aliphatique choisie dans le groupe constitué par la dialcanolamine, la trialcanolamine, et la di(alkyl- ou alcényl-C₈ à C₂₂)méthylamine ; ou
(C4) un mélange d'au moins deux des (C1), (C2) et (C3).

6. Procédé selon la revendication 5, dans lequel le composant (B) est au moins un élément choisi dans le groupe constitué par l'acide 9-hydroxystéarique, l'acide 10-hydroxystéarique, l'acide 12-hydroxystéarique, l'acide 9,10-dihydroxystéarique, l'alcool laurylique, l'alcool myristylique, l'alcool palmitylique, l'alcool stéarylique et l'alcool béhénylique, et
le composant (C) est (C2a) au moins un élément choisi dans le groupe constitué par les sels de lithium, les sels de sodium et les sels de potassium des acides gras en C₈ à C₃₂ saturés ou non qui peuvent avoir au moins un groupe hydroxyle par molécule, ou
le composant (C) est (C1a) au moins un sel d'ester sulfurique choisi dans le groupe constitué par les sels de sulfate de lauryle, les sels de sulfate de stéaryle, les sels de sulfate d'oléyle, les sels de sulfate de l'éther laurylique de polyoxyéthylène (nombre de moles d'oxyde d'éthylène ajoutées = 2 à 3), les sels de sulfate de l'éther stéarylique de polyoxyéthylène (nombre de moles d'oxyde d'éthylène ajoutées = 2 à 3), les sels de sulfate de l'éther nonylphénylique de polyoxyéthylène (nombre de moles d'oxyde d'éthylène ajoutées = 2 à 3), les sels de sulfate de l'éther dodécylphénylique de polyoxyéthylène (nombre de moles d'oxyde d'éthylène ajoutées = 2 à 3), les sels de sulfate du monolaurate de glycéryle, les sels de sulfate du monostéarate de glycéryle, les sels d'esters sulfuriques des monoéthanolamides d'acide laurique, les sels d'esters sulfuriques des monoéthanolamides d'acide stéarique et les sels d'esters sulfuriques des monoéthanolamides d'acide oléique, dans lesquels les sels d'esters sulfuriques ou les sels de sulfate sont les sels de lithium, les sels de sodium et les sels de potassium.

7. Procédé selon la revendication 6, dans lequel le composant (C) est au moins un élément choisi dans le groupe constitué par le laurylsulfate de sodium, le laurylsulfate de potassium, le stéarate de sodium, le stéarate de potassium, le 12-hydroxystéarate de sodium et le 12-hydroxystéarate de potassium.

8. Procédé selon l'une quelconque des revendications 5 à 7, dans lequel le rapport en poids du composant (B) au composant (C) est de 1/0,2 à 5.

9. Composition de diacétal granulaire ou pulvérulente, dans laquelle le transfert d'odeur et de goût provenant du diacétal est supprimé ;
la composition comprenant les composants (A), (B) et (C), où le composant (A) est au moins un diacétal représenté par la formule (1) 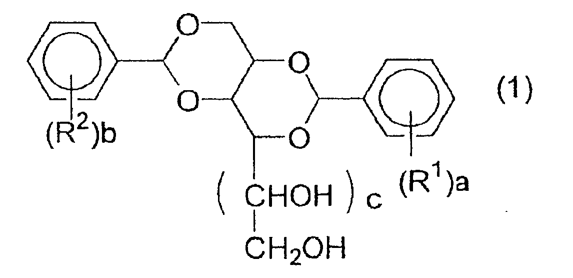 dans laquelle R¹ et R² sont identiques ou différents et chacun représente un atome d'hydrogène, un groupe alkyle en C₁ à C₄, un groupe alcoxy en C₁ à C₄, un groupe alcoxycarbonyle en C₁ à C₄ ou un atome d'halogène ; a et b représentent chacun un nombre entier de 1 à 5 ; c est 0 ou 1 ; quand a vaut 2, les deux groupes R¹ pris ensemble avec le cycle benzénique auquel ils sont liés peuvent former un cycle tétraline ; et quand b vaut 2, les deux groupes R² pris ensemble avec le cycle benzénique auquel ils sont liés peuvent former un cycle tétraline ;
le composant (B) est au moins un élément choisi dans le groupe constitué par :
(B1) les alcools aliphatiques en C₆ à C₃₂ saturés ou non ; et
(B2) les acides carboxyliques aliphatiques en C₈ à C₃₂ saturés ou non ayant au moins un groupe hydroxyle par molécule, et
le composant (C) est
(C1) au moins un tensioactif anionique choisi dans le groupe constitué par les sels d'esters sulfuriques des alcools aliphatiques en C₆ à C₃₀ saturés ou non, les sels d'esters sulfuriques des éthers d'alkyle (C₈ à C₂₂) ou d'alcényle (C₈ à C₂₂) de polyoxyéthylène dans lesquels le nombre de moles d'oxyde d'éthylène ajoutées est de 1 à 8, les sels d'esters sulfuriques des éthers d'alkyl-(C₈ à C₂₂)-phényle (C₈ à C₂₂) de polyoxyéthylène dans lesquels le nombre de moles d'oxyde d'éthylène ajoutées est de 1 à 10, les sels d'esters sulfuriques des esters partiels d'acides gras de polyols formés à partir d'un polyol en C₃ à C₆ et d'un acide gras en C₈ à C₂₂ saturé ou non et les sels d'esters sulfuriques des amides de monoalcanol (C₂ à C₆) d'acides gras en C₈ à C₂₂ saturés ou non, dans lesquels les sels d'esters sulfuriques sont les sels de lithium, les sels de sodium, les sels de potassium et les sels d'ammonium ;
(C2) au moins un élément choisi dans le groupe constitué par les sels de métal alcalin des acides gras en C₈ à C₃₂ saturés ou non qui peuvent avoir au moins un groupe hydroxyle par molécule ;
(C3) au moins une amine aliphatique choisie dans le groupe constitué par la dialcanolamine, la trialcanolamine et la di(alkyl- ou alcényl-C₈ à C₂₂)méthylamine ; ou
(C4) un mélange d'au moins deux des (C1), (C2) et (C3).

10. Composition de diacétal selon la revendication 9, dans laquelle, sur la base de la quantité totale des composants (A), (B) et (C), le composant (B) est présent en une proportion de 0,1 à 5 % en poids et le composant (C) est présent en une proportion de 0,1 à 5 % en poids.

11. Composition de diacétal selon la revendication 10, dans laquelle le rapport en poids du composant (B) au composant (C) est de 1/0,2 à 5.

12. Agent de nucléation pour résine polyoléfinique comprenant la composition de diacétal selon l'une quelconque des revendications 9 à 11, dans lequel le transfert d'odeur et de goût provenant du diacétal est supprimé.

13. Composition de résine polyoléfinique comprenant l'agent de nucléation pour résine polyoléfinique selon la revendication 12 et une résine polyoléfinique, dans laquelle le transfert d'odeur et de goût provenant du diacétal est supprimé.

14. Composition de résine polyoléfinique selon la revendication 13, dans laquelle l'agent de nucléation pour résine polyoléfinique selon la revendication 12 est présent en une quantité de 0,05 à 3 parties en poids pour 100 parties en poids de la résine polyoléfinique.

15. Produit moulé en résine polyoléfinique préparé par moulage de la composition de résine polyoléfinique selon les revendications 13 ou 14, dans lequel le transfert d'odeur et de goût provenant du diacétal est supprimé.

16. Récipient ou matériau d'emballage pour aliments, cosmétiques ou médicaments comprenant le produit moulé en résine polyoléfinique selon la revendication 15, dans lequel le transfert d'odeur et de goût provenant du diacétal est supprimé.

17. Procédé permettant de supprimer l'odeur provenant d'un diacétal lors du moulage d'une résine polyoléfinique, comprenant le mélange de l'agent de nucléation selon la revendication 12 avec une résine polyoléfinique et le moulage d'une composition de résine obtenue.

18. Procédé permettant de supprimer le transfert d'odeur et de goût provenant d'un diacétal à un contenu (tel que des aliments, des cosmétiques et des médicaments), **caractérisé en ce qu'**il comprend le placement du contenu dans un matériau d'emballage ou un récipient préparé par mélange de l'agent de nucléation selon la revendication 12 avec une résine polyoléfinique et le moulage d'une composition de résine obtenue.
